(19) Europäisches Patentamt
European Patent Office
Office européen des brevets

(11) EP 2 981 539 B1

(12) **FASCICULE DE BREVET EUROPEEN**

(45) Date de publication et mention de la délivrance du brevet:
**05.09.2018 Bulletin 2018/36**

(21) Numéro de dépôt: **14719108.4**

(22) Date de dépôt: **01.04.2014**

(51) Int Cl.:
***C07F 5/02*** *(2006.01)* ***C07B 59/00*** *(2006.01)*
***G01N 33/60*** *(2006.01)*

(86) Numéro de dépôt international:
**PCT/IB2014/060356**

(87) Numéro de publication internationale:
**WO 2014/162266 (09.10.2014 Gazette 2014/41)**

(54) **PROCÉDÉ DE PRÉPARATION DE COMPOSES OXYBORANES**

VERFAHREN ZUR HERSTELLUNG VON OXYBORANVERBINDUNGEN

METHOD FOR PREPARING OXYBORANE COMPOUNDS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **03.04.2013 FR 1352996**

(43) Date de publication de la demande:
**10.02.2016 Bulletin 2016/06**

(73) Titulaire: **Commissariat à l'Énergie Atomique et aux Énergies Alternatives**
**75015 Paris (FR)**

(72) Inventeurs:
- **CANTAT, Thibault**
  **F-92130 Issy Les Moulineaux (FR)**
- **GOMES, Christophe**
  **F-92160 Antony (FR)**
- **BLONDIAUX, Enguerrand**
  **F-91400 Orsay (FR)**
- **JACQUET, Olivier**
  **F-91400 Orsay (FR)**

(74) Mandataire: **Gevers & Orès**
**41 avenue de Friedland**
**75008 Paris (FR)**

(56) Documents cités:


- **FANG HUANG ET AL: "How Does the Nickel Pincer Complex Catalyze the Conversion of CO 2 to a Methanol Derivative? A Computational Mechanistic Study", INORGANIC CHEMISTRY, vol. 50, no. 8, 18 avril 2011 (2011-04-18) , pages 3816-3825, XP055081618, ISSN: 0020-1669, DOI: 10.1021/ic200221a**
- **GABRIEL MÉNARD ET AL: "Room Temperature Reduction of CO 2 to Methanol by Al-Based Frustrated Lewis Pairs and Ammonia Borane", JOURNAL OF THE AMERICAN CHEMICAL SOCIETY, vol. 132, no. 6, 17 février 2010 (2010-02-17), pages 1796-1797, XP055081620, ISSN: 0002-7863, DOI: 10.1021/ja9104792 cité dans la demande**
- **SÉBASTIEN BONTEMPS ET AL: "Borane-Mediated Carbon Dioxide Reduction at Ruthenium: Formation of C1 and C2 Compounds", ANGEWANDTE CHEMIE INTERNATIONAL EDITION, vol. 51, no. 7, 13 février 2012 (2012-02-13), pages 1671-1674, XP055081601, ISSN: 1433-7851, DOI: 10.1002/anie.201107352 cité dans la demande**
- **SUMIT CHAKRABORTY ET AL: "An Efficient Nickel Catalyst for the Reduction of Carbon Dioxide with a Borane", JOURNAL OF THE AMERICAN CHEMICAL SOCIETY, vol. 132, no. 26, 7 juillet 2010 (2010-07-07), pages 8872-8873, XP055081607, ISSN: 0002-7863, DOI: 10.1021/ja103982t cité dans la demande**
- **RYO SHINTANI ET AL: "Copper-Catalyzed Hydroboration of Carbon Dioxide", ORGANOMETALLICS, vol. 32, no. 8, 3 avril 2013 (2013-04-03), pages 2459-2462, XP055081611, ISSN: 0276-7333, DOI: 10.1021/om400175h**


Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 981 539 B1

## Description

La présente invention concerne un procédé de préparation de composés oxyboranes utilisant le dioxyde de carbone et l'utilisation des composés oxyboranes ainsi obtenus, pour la préparation des dérivés de méthane, en particulier, des dérivés oxygénés, halogénés ou aminés de méthane. Les dérivés de méthane ainsi obtenus, peuvent alors être utilisés dans la fabrication de vitamines, de produits pharmaceutiques, de colles, de fibres acryliques et cuirs synthétiques, de pesticides, et d'engrais, par exemple.

L'invention concerne également un procédé de fabrication de vitamines, de produits pharmaceutiques, de colles, de fibres acryliques, de cuirs synthétiques, de pesticides, et d'engrais, par exemple, comprenant une étape de préparation des dérivés de méthane, en particulier, des dérivés oxygénés, halogénés ou aminés de méthane, à partir de composés oxyboranes obtenus par le procédé selon l'invention.

La présente invention concerne, en outre, un procédé de préparation de composés oxyboranes marqués et leurs utilisations.

L'utilisation du $CO_2$ valorisable comme source de carbone pour la production de consommables chimiques est un défi de premier plan pour diminuer son accumulation atmosphérique mais également pour contrôler notre dépendance en combustibles fossiles.

Le plus grand défi auquel font face les scientifiques et industriels est de recycler le $CO_2$, c'est-à-dire de développer des réactions permettant de produire des composés chimiques comme, par exemple, des combustibles, des polymères plastiques, des médicaments, des détergents, des molécules à hauts tonnages, traditionnellement obtenus par des méthodes pétrochimiques. La difficulté technique réside dans la mise au point de réactions chimiques qui permettent de fonctionnaliser le $CO_2$ tout en réduisant le centre carboné (i.e. en substituant les liaisons C-O du $CO_2$ par des liaisons C-H ou C-C).

La réduction catalytique du $CO_2$ en acide formique HCOOH, formaldéhyde $H_2CO$, méthanol $CH_3OH$ et méthane $CH_4$ suscite un intérêt grandissant dans la recherche de nouveaux carburants synthétiques. Dans ce contexte, les grands procédés de réduction peuvent être classés selon la nature du réducteur utilisé, comme indiqué aux paragraphes 1 à 4, ci-dessous. L'utilisation de réducteurs puissants, tels que les métaux alcalins (Li, Na, K) ou les hydrures métalliques (hydrure d'aluminium, borohydrures, etc.), est écartée car ces réactifs conduisent à des réactions fortement exothermiques en présence de $CO_2$ et ne permettent donc pas d'assurer un bilan énergétique favorable lors de la réduction du dioxyde de carbone.

*1. Méthodes électro- et photoélectro-chimiques*

L'utilisation d'électrons fournis par un montage d'électrolyse pour réduire le $CO_2$ reste un domaine de recherche très dynamique et motivé par l'espoir de trouver des catalyseurs efficaces et sélectifs, permettant par exemple de réduire sélectivement le $CO_2$ en présence de protons, tout en évitant la formation de dihydrogène $H_2$ (E. E. Benson, C. P. Kubiak, A. J. Sathrum, J. M. Smieja, Chem. Soc. Rev. 2009, 38, 89). Des procédés de photoélectro-réduction sont également à l'étude (Y. Izumi, Coord. Chem. Rev. 2013, 257, 171).

*2. Hydrogénation du $CO_2$*

La réaction entre le $CO_2$ et le dihydrogène peut conduire à la formation d'acide formique (en présence d'une base), de méthanol ou de méthane. Des catalyseurs moléculaires (dits homogènes) et des catalyseurs hétérogènes ont été décrits pour faciliter cette réaction (P. G. Jessop, T. Ikariya, R. Noyori, Chem. Rev. 1995, 95, 259 ; W. Wang, S. Wang, J. Gong, **2011**, 3703).

*3. Hydrosilylation du $CO_2$*

La réaction entre le $CO_2$ et des hydrosilanes (caractérisés par la présence d'une liaison Si-H) permet de réduire le $CO_2$ en formoxysilane, bis(silyl)acétals et méthoxysilanes qui peuvent conduire après hydrolyse à l'acide formique HCOOH, au formaldéhyde $H_2CO$ et au méthanol $CH_3OH$, respectivement (S. N. Riduan, Y. G. Zhang, J. Y. Ying, Angew. Chem. Int. Ed. 2009, 48, 3322 ; A. Berkefeld, W. E. Piers, M. Parvez, J. Am. Chem. Soc. 2010, 132, 10660). Certains catalyseurs permettent également de réduire le $CO_2$ directement en méthane (T. Matsuo, H. Kawaguchi, J. Am. Chem. Soc. 2006, 128). Dans ces réactions, des siloxanes et silanols sont formés comme sous-produits.

*4. Hydroboration du $CO_2$*

La réaction entre le $CO_2$ et un hydroborane de formule (**I**) est dite réaction d'hydroboration du $CO_2$. Cette transformation nécessite l'emploi d'un catalyseur. Trois systèmes catalytiques différents sont connus à ce jour. Ils sont

détaillés ci-dessous.

- Le groupe de Hairong Guan (University of Cincinnatti, USA) a mis au point le premier catalyseur d'hydroboration du $CO_2$ en 2010 (S. Chakraborty, J. Zhang, J. A. Krause, H. R. Guan, J. Am. Chem. Soc. 2010, 132, 8872 ; S. Chakraborty, Y. J. Patel, J. A. Krause, H. R. Guan, Polyhedron 2012, 32, 30 ; S. Chakraborty, J. Zhang, Y. J. Patel, J. A. Krause, H. R. Guan, Inorg. Chem. 2013, 52, 37). Il s'agit d'un complexe de nickel qui permet d'effectuer la réduction du $CO_2$ en méthoxyborane. Le formoxyborane est observé comme intermédiaire réactionnel. Les hydroboranes utilisés sont le catécholborane (catBH), le 9-borabicyclo[3.3.1]nonane (9-BBN) et le pinacholborane (pinBH). Le catalyseur opère à la température ambiante et en présence d'1 bar de $CO_2$. Avec le catécholborane, le nombre de rotation du catalyseur (en anglais Turn-Over Number ou TON, défini ci-dessous) est de 495 à 25 °C et sa fréquence de rotation (en anglais Turn-Over Frequency ou TOF, définie ci-dessous) de 495 $h^{-1}$. Cette réaction est indiquée dans le schéma 1 ci-dessous.

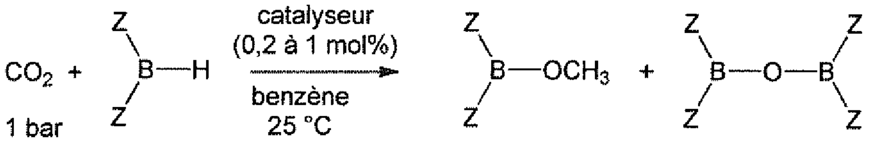


(I) (IV) (V)

catalyseur =

R = t-Bu, i-Pr,

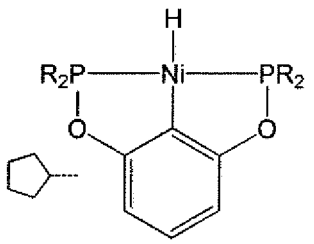

pour R=t-Bu:
TON = 495
TOF = 495 $h^{-1}$
à 25 °C avec catécholborane

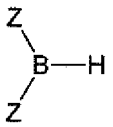

=

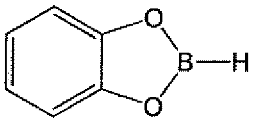

catécholborane (catBH)

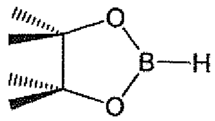

pinacholborane (pinBH)

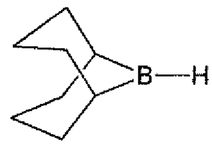

9-borabicyclo[3.3.1]nonane (9-BBN)

Schéma 1

Dans le schéma ci-dessus ainsi que dans la suite de l'exposé, le TON et la TOF se définissent comme suit :

$$TON = \frac{\text{quantité de borane } (R^1R^2BH) \text{ en fin de réaction}}{\text{quantité de borane } (R^1R^2BH) \text{ en début de réaction}} \times \frac{100}{\text{charge catalytique en mol\%}}$$

$$TOF = \frac{\text{quantité de borane en fin de réaction}}{\text{quantité de borane en début de réaction}} \times \frac{100}{\text{charge catalytique en mol\%}} \times \frac{1}{\text{temps de réaction en heures}}$$

Ainsi, plus le TON et la TOF sont élevés et plus le catalyseur est efficace.

- En 2012, le groupe de Sylviane Sabo-Etienne (CNRS, Toulouse, France) a décrit un catalyseur basé sur un complexe hydrure de ruthénium pour la réaction d'hydroboration du $CO_2$ (S. Bontemps, L. Vendier, S. Sabo-Etienne, Angew. Chem. Int. Ed. 2012, 51, 1671). Les auteurs ont montré que la réaction d'hydroboration du $CO_2$ pouvait conduire à des intermédiaires de type bis(boryl)acétals et boroxyméthylformiate ($R^1R^2B\text{-}OCH_2OCHO$). Ces intermédiaires n'ont pas été isolés.
  Seul le pinacholborane a été utilisé et une charge importante de catalyseur a été utilisée (10 mol%). Dans ces

conditions, l'activité du catalyseur est faible et la formation de méthoxyborane nécessite 22 jours de réaction à la température ambiante ou 5 h à 70 °C. Cette réaction est indiquée dans le schéma 2 ci-dessous.

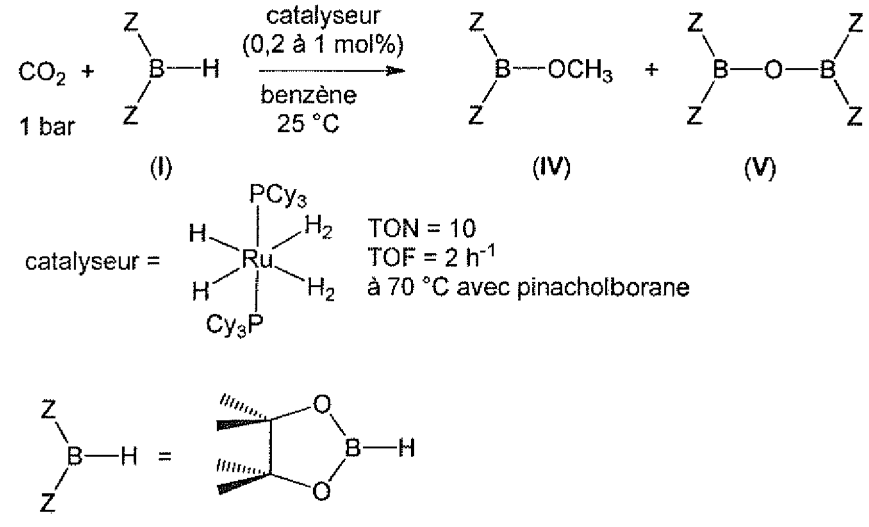


pinacholborane (pinBH)

Schéma 2

- En 2012, le groupe de Douglas W. Stephan (University of Toronto, Canada) a décrit un catalyseur à base de ruthénium pour l'hydroboration du $CO_2$ (M. J. Sgro, D. W. Stephan, Angew. Chem. Int. Ed. 2012, 51, 11343). Le catécholborane et le 9-borabicyclo[3.3.1]nonane ont été utilisés comme réactifs. Ils ne montrent pas de différence de réactivité. La réaction est lente à 50 °C avec une charge de catalyseur de 1,0 mol%. Cette réaction est indiquée dans le schéma 3 ci-dessous.

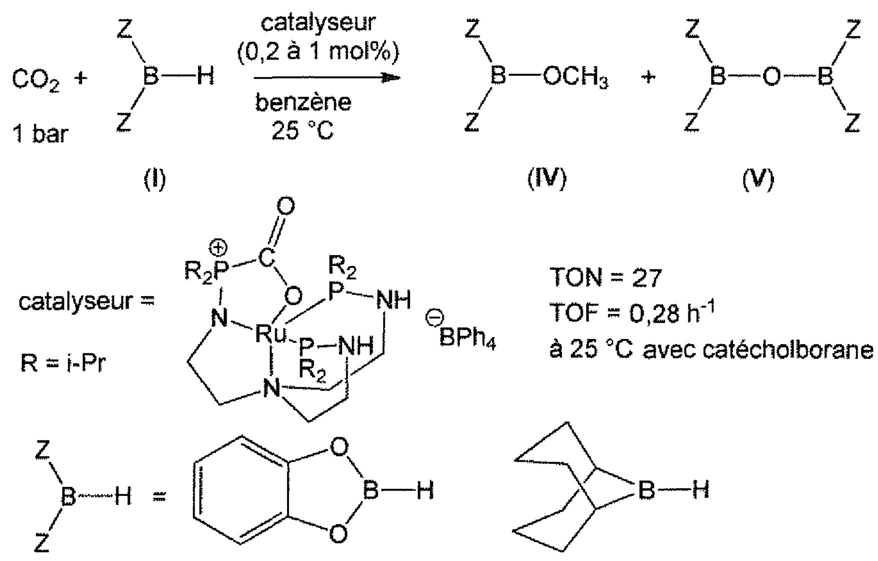


catécholborane (catBH) 9-borabicyclo[3.3.1]nonane (9-BBN)

Schéma 3

- Les transformations faisant intervenir des promoteurs réactionnels (tels que des mélanges $Mes_3P/AlCl_3$ ou $Mes_3P/AlBr_3$ (Mes=mésityle)) en quantités stoechiométriques, c'est-à-dire non-catalytiques, ont également été décrites par G. Ménard, D. W. Stephan, J. Am. Chem. Soc., 2010, 132, 1796.

La conversion du $CO_2$ en consommables chimiques comme, par exemple, des dérivés de méthane, en particulier, des dérivés oxygénés, halogénés ou aminés de méthane notamment l'acide formique, le formaldéhyde et le méthanol, le méthane, l'halogénure de méthyle et la méthylamine, par une réaction d'hydroboration du $CO_2$ suscite un intérêt grandissant. La réaction du $CO_2$ avec un hydroborane se faisant en deux étapes, conduit à des intermédiaires synthétiques intéressants de type formoxy borane ($R^1R^2B$-O-CHO), méthoxyborane ($R^1R^2B$-O-$CH_3$) ou bis(boryl)acétals ($(R^1R^2B\text{-}O)_2CH_2$). Ces intermédiaires qui peuvent également être désignés plus généralement comme des « composés oxyboranes » du fait de la présence de « $R^1R^2B$-O- » dans ces composés, sont stables et se prêtent aisément à différents types de réaction pour conduire à des composés chimiques variés comme l'acide formique, le formaldéhyde, le méthanol, le méthane, l'halogénure de méthyle, la méthylamine, etc.

Cependant, compte tenu de la grande stabilité thermodynamique du dioxyde de carbone, sa conversion en composés oxyboranes doit nécessairement faire appel à des catalyseurs efficaces de manière à favoriser le bilan thermodynamique de cette transformation chimique.

Par ailleurs, la réaction d'hydroboration nécessite l'emploi d'un catalyseur efficace car en son absence, le produit résultant de cette transformation chimique ne peut être obtenu de façon mesurable dans un délai court (moins d'une semaine) et à une température inférieure à 150°C.

Or, à ce jour, les réactions d'hydroboration du $CO_2$ mettent en jeu un nombre limité de catalyseurs qui, de surcroît, sont essentiellement des complexes de métaux de transition souvent coûteux et/ou toxiques comme le nickel ou le ruthénium.

F. Huang et al. (Inorganic Chemistry, 2011, vol. 50, no. 8, pages 3816-3825) enseigne l'utilisation de catalyseurs contenant du nickel dans la réduction du $CO_2$ avec le catecholborane et le compare à un système théorique NHC/$CO_2$/HBcat dans lequel NHC représente un carbène. Ce document évoque le problème lié à la libération de NHC dans un système NHC/$CO_2$/HBcat du fait de la stabilité de l'intermédiaire thermodynamiquement (NHC-HCOOBcat) et indique que HBcat ne peut être une bonne source d'hydrogène pour la réduction de $CO_2$ catalysée par NHC.

Dans le cadre de la conversion du $CO_2$ par une réaction d'hydroboration, d'abord en « composés oxyboranes » puis en consommables chimiques comme, par exemple, des dérivés de méthane, en particulier, des dérivés oxygénés, halogénés ou aminés de méthane notamment l'acide formique, le formaldéhyde, le méthanol, le méthane, l'halogénure de méthyle et la méthylamine, le défi technique à relever est de mettre au point des catalyseurs efficaces qui s'affranchissent des problèmes de toxicité et de coûts généralement associés à l'utilisation de catalyseurs métalliques connus, notamment des catalyseurs à base de métaux précieux.

Il existe donc un réel besoin d'un catalyseur permettant la conversion du $CO_2$ et d'un hydroborane en composés oxyboranes, par une réaction d'hydroboration, qui soit efficace (capable d'augmenter la vitesse de la conversion de la $CO_2$ même en faible quantité), sélectif (favorisant la production du produit désiré par rapport aux produits secondaires), peu coûteux et/ou peu toxique comparé aux catalyseurs connus pour la conversion du $CO_2$ en composés oxyboranes par ce type de réaction.

En particulier, il existe un réel besoin d'un catalyseur, tel que défini ci-dessus, qui ne contienne pas :

- de métaux alcalinoterreux du Groupe IIA du Tableau Périodique des Eléments (comme le magnésium et le calcium) ;
- de métaux de transition du Groupe IB à VIIIB du Tableau Périodique des Eléments (comme le nickel, le fer, le cobalt, le zinc, le cuivre, le rhodium, le ruthénium, le platine, le palladium, l'iridium) ;
- de terres rares dont le numéro atomique est compris entre 57 et 71 (comme le lanthane, le cérium, le praséodyme, le néodyme) ; ou
- d'actinides dont le numéro atomique est compris entre 89 et 103 (comme le thorium, l'uranium).

Par ailleurs, les composés oxyboranes, incorporant des radioisotopes et/ou isotopes stables et capables de se transformer en différents composés chimiques marqués comme l'acide formique, le formaldéhyde, le méthanol, le méthane, l'halogénure de méthyle, la méthylamine, etc. présentent un intérêt particulier dans de nombreux domaines comme, par exemple, dans les sciences du vivant (étude/élucidation de mécanismes enzymatiques, de mécanismes biosynthétiques, en biochimie, etc.), les sciences de l'environnement (traçage de déchets, etc.), la recherche (étude/élucidation de mécanismes réactionnels) ou encore la recherche et le développement de nouveaux produits pharmaceutiques et thérapeutiques. Ainsi, développer un procédé pour la préparation de composés oxyboranes marqués répondant aux exigences indiquées ci-dessus, peut répondre à un besoin réel.

Il existe, donc, un réel besoin de disposer d'un procédé qui permette de préparer des composés oxyboranes marqués incorporant des radioisotopes et/ou isotopes stables, à partir de réactifs marqués comme par exemple le $CO_2$ et/ou un hydroborane marqué(s).

La présente invention a précisément pour but de répondre à ces besoins en fournissant un procédé de préparation de composés oxyboranes de formule (I) selon la revendication 1 :

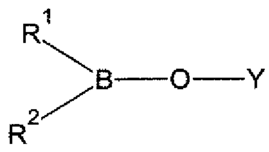

(I)

dans laquelle

- $R^1$ et $R^2$ représentent, indépendamment l'un de l'autre, un atome d'hydrogène, un atome d'halogène, un groupe alkyle linéaire, ramifié ou cyclique, un groupe alcényle, un groupe alcynyle, un groupe aryle, un groupe hétéroaryle, un hétérocycle, un groupe silylé, un groupe siloxy, un groupe amino, un groupe alkoxy, lesdits groupes alkyle, alcényle, alcynyle, aryle, hétéroaryle, hétérocycle, silylé, siloxy, amino et alkoxy étant éventuellement substitués, ou
- $R^1$ et $R^2$, pris ensemble avec l'atome de bore auquel ils sont liés, forment un hétérocycle éventuellement substitué,
- Y représente -CHO, $-CH_2-O-BR^1R^2$ avec $R^1$ et $R^2$ tels que définis ci-dessus, $-CH_3$,
- $R^1$, $R^2$ et Y comportent éventuellement, indépendamment l'un de l'autre, un H, C, N, O, F, Si et/ou S tels que définis ci-dessous,

- H représente un atome d'hydrogène ($^1H$), le deutérium ($^2H$) ou le tritium ($^3H$),
- C représente un atome de carbone ($^{12}C$), un isotope $^{11}C$, $^{13}C$ ou $^{14}C$,
- N représente un atome d'azote ($^{14}N$), un isotope $^{15}N$,
- O représente un atome d'oxygène ($^{16}O$), un isotope $^{18}O$,
- F représente un atome de fluor ($^{19}F$), un isotope $^{18}F$,
- Si représente un atome de silicium ($^{28}Si$), un isotope $^{29}Si$ ou $^{30}Si$,
- S représente un atome de soufre ($^{32}S$), un isotope $^{33}S$, $^{34}S$ ou $^{36}S$,

caractérisé en ce que l'on fait réagir un hydroborane de formule (II) dans laquelle $R^1$, $R^2$ et H sont tels que définis ci-dessus

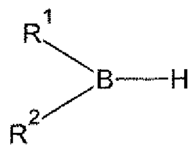

(II)

avec du $CO_2$ dans lequel C et O sont tels que définis ci-dessus, et
en présence d'un catalyseur choisi dans le groupe constitué de

i. bases organiques choisies parmi les bases organiques azotées, les bases organiques phosphorées, , ou les bases organiques oxygénées

- les bases organiques azotées étant des amines secondaires ou tertiaires choisies parmi, le triazabicyclodécène (TBD), le N-méthyltriazabicyclodécène (MeTBD), le 1,8-diazabicyclo[5.4.0]undéc-7-ène (DBU), la triméthylamine, la triéthyleamine, la pipéridine, la 4-diméthylaminopyridine (DMAP), le 1,4-diazabicyclo[2.2.2]octane (DABCO), la proline, le phénylalanine, un sel de thiazolium, la *N*-diisopropyléthylamine (DIPEA ou DIEA), l'arginine ; les phosphazènes choisis parmi, par exemple, le tert-butylimino-tris(diméthylamino)phosphorane (P1-t-Bu), le tert-butylimino-tri(pyrrolidino)phosphorane (BTPP), le tétraméthyl(tris(diméthylamino)phosphoranylidène)phosphorictriamid-Et-imine (P2-Et), le tert-octylimino-tris(diméthylamino)phosphorane (P1-t-Oct) et le 1-tert-butyl-4,4,4-tris(diméthylamino)-2,2-bis[tris(diméthylamino)-phosphoranylidenamino]-2λ5,4λ5-caténadi(phosphazène) (P4-Bu) ;
- les bases organiques phosphorées étant des alkyles ou aryles phosphines choisis parmi le triphénylphosphine, le 2,2'-bis(diphénylphosphino)-1,1'-binaphthyle (BINAP), le triisopropylphosphine, le 1,2-bis(diphénylphosphino)éthane (dppe), la tricyclohexylphosphine ($PCy_3$) ; les alkyle et aryle phosphonates choisis parmi le diphénylphosphate, le triphénylphosphate (TPP), le tri(isopropylphényl)phosphate (TIPP), le crésyldiphenyl phosphate (CDP), le tricrésylphosphate (TCP) ; les alkyle et aryle phosphates choisis parmi le di-n-butylphosphate (DBP), le tris-(2-éthylhexyl)-phosphate, le triéthyl phosphate ; les alkyle et aryle phosphinites et phosphonites choisis parmi les méthyldiphénylphosphinite et méthyldiphénylphosphonite ; les

aza-phosphines choisies parmi le 2,8,9-triisopropyl-2,5,8,9-tetraaza-1-phosphabicyclo[3.3.3]undecane ($BV^{Me}$) et le 2,8,9-triisobutyl-2,5,8,9-tetraaza-1-phosphabicyclo[3.3.3]undecane ($BV^{iBu}$) ; ou

- les bases oxygénées choisies parmi le peroxyde d'hydrogène, le peroxyde de benzoyle, l'oxyde de pyridine (PyO), l'oxyde de N-méthylmorpholine ou le 1-λ-oxidanyl-2,2,6,6-tétraméthylpipéridine ;

ii. composés organiques ou inorganiques de bore choisis parmi le $BF_3$, le $BF_3(Et_2O)$, le $BCl_3$, le triphényl hydroborane, le tricyclohexyl hydroborane, le $B(C_6F_5)_3$, le B-méthoxy-9-borabicyclo[3.3.1]nonane, le B-benzyl-9-borabicyclo[3.3.1]nonane (B-méthoxy-9BBN), le Me-TBD-$BBN^+$ $I^-$, le Me-TBD-$BBN^+$ $CF_3SO_3^-$, le $(TDB\text{-}BBN)_2$, le TBD-BBN-$CO_2$ ou le TBD-BBN-BBN ; ou

iii. composés organiques ou inorganiques de l'aluminium choisis parmi $AlCl_3$, $AlBr_3$, l'isopropoxyde d'aluminium ou l'éthanoate d'aluminium.

Ainsi, le procédé de l'invention permet de préparer aussi bien des composés oxyboranes de formule (I) non marqués que des composés oxyboranes de formule (I) marqués.

Le procédé de l'invention a également l'avantage de permettre de convertir le $CO_2$ en composés oxyboranes avec un grand choix de catalyseurs. Les catalyseurs utilisés dans le procédé de l'invention s'affranchissent des problèmes de toxicité et de coûts associés généralement à l'utilisation de catalyseurs métalliques dont le métal est

- un métal alcalinoterreux du Groupe IIA du Tableau Périodique des Eléments (comme le magnésium et le calcium) ;
- un métal de transition du Groupe IB à VIIIB du Tableau Périodique des Eléments (comme le nickel, le fer, le cobalt, le zinc, le cuivre, le rhodium, le ruthénium, le platine, le palladium, l'iridium) ;
- une terre rare dont le numéro atomique est compris entre 57 et 71 (comme le lanthane, le cérium, le praséodyme, le néodyme) ; ou
- une actinide dont le numéro atomique est compris entre 89 et 103 (comme le thorium, l'uranium).

Selon les conditions, le procédé de l'invention, les composés oxyboranes peuvent être obtenus sous forme d'un mélange de composés de formules (I) ou avec une bonne sélectivité (pouvant atteindre 100% en un seul type de composé oxyborane de formule (I)).

Le procédé de l'invention peut conduire aux composés oxyborane de formule (I) avec un bon voire excellent rendement (allant de 50% à 100%, par exemple).

Dans le cadre de la présente invention, le rendement est calculé par rapport à la quantité d'hydroborane de formule (II) introduit initialement, sur la base de la quantité de composé oxyborane de formule (I) isolé :

Rendement= n(oxyborane)/(n(oxyborane)+n(hydroborane)) n étant la quantité de matière

Dans le cadre de la présente invention, la sélectivité se rapporte à la nature des produits oxyboranes de formule (I) formés à partir de l'hydroborane de formule (II).

Pour que le procédé de l'invention puisse conduire à l'obtention d'un composé oxyborane de formule (I), une combinaison judicieuse et adéquate d'hydroboranes de formule (II) et de catalyseurs est essentielle. Il est en particulier nécessaire que l'hydroborane de formule (II) et le catalyseur soient choisis en tenant compte notamment de leur encombrement stérique respectif, du caractère réducteur de l'hydroborane, du caractère nucléophile du catalyseur, de leur solubilité dans le milieu réactionnel.

On entend par « alkyle », au sens de la présente invention, un radical carboné linéaire, ramifié ou cyclique, saturé ou insaturé, éventuellement substitué, comprenant 1 à 12 atomes de carbone. A titre d'alkyle saturé, linéaire ou ramifié, on peut citer par exemple les radicaux méthyle, éthyle, propyle, butyle, pentyle, hexyle, octyle, nonyle, décyle, undécyle, dodécanyle et leurs isomères ramifiés. Comme alkyle cylique, on peut citer les radicaux cyclopropyle, cyclobutyle, cyclopentyle, cyclohexyle, bicylco[2,1,1] hexyle, bicyclo[2,2,1] heptyle. Comme alkyles cycliques insaturés, on peut citer par exemple le cyclopentényle, le cyclohexényle. Les alkyles insaturés appelés également « alcényle » ou « alcynyle » contiennent respectivement au moins une double ou une triple liaison. A ce titre, on peut citer, par exemple, les radicaux éthylényle, propylényle, butényle, pentényle, hexényle, acétylényle, propynyle, butynyle, pentynyle, hexynyle et leurs isomères ramifiés. Le groupe alkyle, au sens de l'invention incluant les groupes alcényle et alcynyle, peut être éventuellement substitué par un ou plusieurs groupes hydroxyle ; un ou plusieurs groupes alkoxy ; un ou plusieurs atomes d'halogène choisis parmi les atomes de fluor, chlore, brome et iode ; un ou plusieurs groupes nitro ($-NO_2$) ; un ou plusieurs groupes nitrile (-CN) ; un ou plusieurs groupes aryle, avec les groupes alkoxy et aryle tels que définis dans le cadre de la présente invention.

Le terme « aryle » désigne de manière générale un substituant aromatique cyclique comportant de 6 à 20 atomes de carbone. Dans le cadre de l'invention le groupe aryle peut être mono- ou polycyclique. A titre indicatif, on peut citer les groupes phényle, benzyle et naphtyle. Le groupe aryle peut être éventuellement substitué par un ou plusieurs groupes hydroxyle, un ou plusieurs groupes alkoxy, un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes alkyle, avec les groupes alkoxy et alkyle tels que définis dans le cadre de la présente invention.

Le terme « hétéroaryle » désigne de manière générale un substituant aromatique mono- ou polycyclique comportant de 5 à 10 membres dont au moins 2 atomes de carbone, et au moins un hétéroatome choisi parmi l'azote, l'oxygène, le bore, le silicium, le phosphore ou le soufre. Le groupe hétéroaryle peut être mono- ou poly- cyclique. A titre indicatif, on peut citer les groupes furyle, benzofuranyle, pyrrolyle, indolyle, isoindolyle, azaindolyle, thiophényle, benzothiophényle, pyridyle, quinolinyle, isoquinolyle, imidazolyle, benzimidazolyle, pyrazolyle, oxazolyle, isoxazolyle, benzoxazolyle, thiazolyle, benzothiazolyle, isothiazolyle, pyridazinyle, pyrimidilyle, pyrazinyle, triazinyle, cinnolinyle, phtalazinyle, quinazolinyle. Le groupe hétéroaryle peut être éventuellement substitué par un ou plusieurs groupes hydroxyle, un ou plusieurs groupes alkoxy, un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes aryle, un ou plusieurs groupes alkyle, avec les groupes alkyle, alkoxy et aryle tels que définis dans le cadre de la présente invention.

Le terme « alkoxy » signifie un groupe alkyle, tel que défini ci-dessus, lié par un atome d'oxygène (-O-alkyle).

Le terme « hétérocycle » désigne de manière générale un substituant mono- ou polycyclique, comportant de 5 à 10 membres, saturé ou instauré, contenant de 1 à 4 hétéroatomes choisis indépendamment l'un de l'autre, parmi l'azote, l'oxygène, le bore, le silicium, le phosphore ou le soufre. A titre indicatif, on peut citer le borolane, le borole, le borinane, le 9-borabicyclo[3.3.1]nonane (9-BBN), le 1,3,2-benzodioxaborole (catecholborane ou catBH), pinacholborane (pinBH), les substituants morpholinyle, pipéridinyle, pipérazinyle, pyrrolidinyle, imidazolidinyle, imidazolinyle, pyrazolidinyle, tétrahydrofuranyle, tétrahydropyranyle, thianyle, oxazolidinyle, isoxazolidinyle, thiazolidinyle, isothiazolidinyle. L'hétérocycle peut être éventuellement substitué par un ou plusieurs groupes hydroxyle, un ou plusieurs groupes alkoxy, un ou plusieurs groupes aryle, un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes alkyle, avec les groupes alkyle, alkoxy et aryle tels que définis dans le cadre de la présente invention.

Par atome d'halogène, on entend un atome choisi parmi les atomes de fluor, chlore, brome ou iode.

Par groupe « silylé », on entend un groupe de formule $[-Si(X)_3]$ dans lequel X est choisi parmi un atome d'hydrogène, un ou plusieurs atomes d'halogène choisis parmi les atomes de fluor, chlore, brome ou iode, un ou plusieurs groupes alkyle, un ou plusieurs groupes alkoxy, un ou plusieurs groupes siloxy, un ou plusieurs groupes aryle, avec les groupes alkyle, alkoxy et aryle tels que définis dans le cadre de la présente invention.

Par groupe « siloxy », on entend un groupe silylé, tel que défini ci-dessus, lié par un atome d'oxygène ($-O-Si(X)_3$).

Par groupe « amino », on entend un groupe de formule $-NR^3R^4$, dans laquelle :

- $R^3$ et $R^4$ représentent, indépendamment l'un de l'autre, un atome d'hydrogène, un groupe alkyle, un groupe alcényle, un groupe alcynyle, un groupe aryle, un groupe hétéroaryle, un hétérocycle, un groupe silylé, un groupe siloxy, avec les groupes alkyle, alcényle, alcynyle, aryle, hétéroaryle, hétérocycle, silylé, siloxy, tels que définis dans le cadre de la présente invention ; ou
- $R^3$ et $R^4$, pris ensemble avec l'atome d'azote auquel ils sont liés, forment un hétérocycle éventuellement substitué par un ou plusieurs groupes hydroxyle ; un ou plusieurs groupes alkyle ; un ou plusieurs groupes alkoxy ; un ou plusieurs atomes d'halogène choisis parmi les atomes de fluor, chlore, brome et iode ; un ou plusieurs groupes nitro ($-NO_2$) ; un ou plusieurs groupes nitrile (-CN) ; un ou plusieurs groupes aryle ; avec les groupes alkyle, alkoxy et aryle tels que définis dans le cadre de la présente invention.

Les substituents, radicaux et groupes définis ci-dessus peuvent comporter, éventuellement, du deutérium ($^2H$), du tritium ($^3H$), du $^{11}C$, du $^{13}C$, du $^{14}C$, du $^{15}N$, du $^{18}O$, du $^{18}F$, du $^{29}Si$, du $^{30}Si$, du $^{33}S$, du $^{34}S$ ou du $^{36}S$.

Selon une variante préférée de l'invention, dans le composé oxyborane de formule (I) et dans l'hydroborane de formule (II),

- $R^1$ et $R^2$ représentent, indépendamment l'un de l'autre, un atome d'hydrogène, un groupe alkyle linéaire, ramifié ou cyclique, un groupe aryle, un groupe alkoxy, lesdits groupes alkyle, aryle et alkoxy étant éventuellement substitués, ou
- $R^1$ et $R^2$, pris ensemble avec l'atome de bore auquel ils sont liés forment un hétérocycle éventuellement substitué.

De préférence, dans le composé oxyborane de formule (I) et dans l'hydroborane de formule (II),

- $R^1$ et $R^2$, représentent, indépendamment l'un de l'autre, un atome d'hydrogène ; un groupe alkyle linéaire, ramifié

ou cyclique choisi parmi les groupes méthyle, éthyle, propyle, butyle, pentyle, hexyle, heptyle, ou leurs isomères ramifiés, cyclohexyle ; un groupe aryle choisi parmi le benzyle ou le phényle ; ou

- $R^1$ et $R^2$, pris ensemble avec l'atome de bore auquel ils sont liés forment un hétérocycle, ledit hétérocycle étant choisi parmi le catBH, le pinBH ou le 9-BBN.

Selon un autre mode de réalisation, l'invention concerne le procédé de préparation de composés oxyboranes de formule (I) selon la revendication 4 :

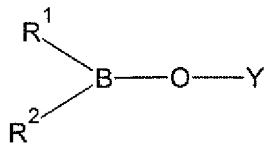

(I)

dans laquelle

- $R^1$ et $R^2$ représentent, indépendamment l'un de l'autre, un atome d'hydrogène, un atome d'halogène, un groupe alkyle linéaire, ramifié ou cyclique, un groupe alcényle, un groupe alcynyle, un groupe aryle, un groupe hétéroaryle, un hétérocycle, un groupe silylé, un groupe siloxy, un groupe amino, un groupe alkoxy, lesdits groupes alkyle, alcényle, alcynyle, aryle, hétéroaryle, hétérocycle, silylé, siloxy, amino et alkoxy étant éventuellement substitués, ou
- $R^1$ et $R^2$, pris ensemble avec l'atome de bore auquel ils sont liés, forment un hétérocycle éventuellement substitué, ledit hétérocycle étant le 9-BBN,
- Y représente -CHO, $-CH_2-O-BR^1R^2$ avec $R^1$ et $R^2$ tels que définis ci-dessus, $-CH_3$,
- $R^1$, $R^2$ et Y comportent éventuellement, indépendamment l'un de l'autre, un H, C, N, O, F, Si et/ou S tels que définis ci-dessous,

  - H représente un atome d'hydrogène ($^1H$), le deutérium ($^2H$) ou le tritium ($^3H$),
  - C représente un atome de carbone ($^{12}C$), un isotope $^{11}C$, $^{13}C$ ou $^{14}C$,
  - N représente un atome d'azote ($^{14}N$), un isotope $^{15}N$,
  - O représente un atome d'oxygène ($^{16}O$), un isotope $^{18}O$,
  - F représente un atome de fluor ($^{19}F$), un isotope $^{18}F$,
  - Si représente un atome de silicium ($^{28}Si$), un isotope $^{29}Si$ ou $^{30}Si$,
  - S représente un atome de soufre ($^{32}S$), un isotope $^{33}S$, $^{34}S$ ou $^{36}S$,

caractérisé en ce que l'on fait réagir un hydroborane de formule (II) dans laquelle $R^1$, $R^2$ et H sont tels que définis ci-dessus

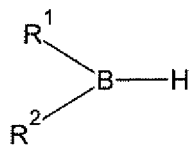

(II)

avec du $CO_2$ dans lequel C et O sont tels que définis ci-dessus, et
en présence d'un catalyseur choisi dans le groupe constitué de bases organiques choisies parmi les bases carbonées, lesdites bases carbonées étant des carbènes N-hétérocycliques issus d'un sel d'imidazolium choisis parmi les sels de 1,3-bis(2,6-diisopropylphenyl)-1H-imidazol-3-ium, 1,3-bis(2,6-diisopropylphenyl)-4,5-dihydro-1H-imidazol-3-ium, 1,3-bis(2,4,6-triméthylphenyl)-1H-imidazol-3-ium, 1,3-bis(2,4,6-triméthylphenyl)-4,5-dihydro-1H-imidazol-3-ium, 4,5-dichloro-1,3-bis(2,6-diisopropylphenyl)-1H-imidazol-3-ium, 1,3-di-tert-butyl-1H-imidazol-3-ium ou carbène ItBu, 1,3-di-tert-butyl-4,5-dihydro-1H-imidazol-3-ium, lesdits sels étant sous la forme de sels de chlorure.

Selon une variante préférée de ce mode de réalisation de l'invention, dans le composé oxyborane de formule (I) et dans l'hydroborane de formule (II), l'invention

- $R^1$ et $R^2$ représentent, indépendamment l'un de l'autre, un atome d'hydrogène, un groupe alkyle linéaire, ramifié ou cyclique, un groupe aryle, un groupe alkoxy, lesdits groupes alkyle, aryle et alkoxy étant éventuellement substitués,

ou

- $R^1$ et $R^2$, pris ensemble avec l'atome de bore auquel ils sont liés, forment un hétérocycle éventuellement substitué, ledit hétérocycle étant le 9-BBN.

De préférence, dans dans le composé oxyborane de formule (I) et dans l'hydroborane de formule (II),
**[0046]** Selon une autre variante préférée de ce mode de réalisation de l'invention, le catalyseur est le 1,3-di-tert-butyl-1H-imidazol-3-ium ou carbène ItBu.
Par catalyseur, au sens de l'invention, on entend tout composé capable de modifier, notamment en augmentant, la vitesse de la réaction chimique à laquelle il participe, et qui est régénéré à la fin de la réaction. Cette définition englobe à la fois les catalyseurs, c'est-à-dire les composés qui exercent leur activité catalytique sans avoir besoin de subir une quelconque modification ou conversion, et les composés (appelés également pré-catalyseurs) qui sont introduits dans le milieu réactionnel et qui y sont convertis en un catalyseur.
Comme déjà indiqué, dans le procédé de l'invention, le catalyseur peut être (i) une base organique choisie parmi les bases organiques azotées, les bases organiques phosphorées, ou les bases organiques oxygénées, avec

- les bases organiques azotées pouvant être des amines secondaires ou tertiaires choisies parmi, par exemple, le triazabicyclodécène (TBD), le N-méthyltriazabicyclodécène (MeTBD), le 1,8-diazabicyclo[5.4.0]undéc-7-ène (DBU), la triméthylamine, la triéthyleamine, la pipéridine, la 4-diméthylaminopyridine (DMAP), le 1,4-diazabicyclo[2.2.2]octane (DABCO), la proline, le phénylalanine, un sel de thiazolium, la *N*-diisopropyléthylamine (DIPEA ou DIEA) l'arginine ; les phosphazènes choisis parmi, par exemple, le tert-butylimino-tris(diméthylamino)phosphorane (P1-t-Bu), le tert-butylimino-tri(pyrrolidino)phosphorane (BTPP), le tétraméthyl(tris(diméthylamino) phosphoranylidène)phosphorictriamid-Et-imine (P2-Et), le tert-octylimino-tris(diméthylamino)phosphorane (P1-t-Oct) et le 1-tert-butyl-4,4,4-tris(diméthylamino)-2,2-bis[tris(diméthylamino)-phosphoranylidenamino]-2λ5,4λ5-caténadi(phosphazène) (P4-Bu) ;
- les bases organiques phosphorées pouvant être des alkyles ou aryles phosphines choisies parmi, par exemple, le triphénylphosphine, le 2,2'-bis(diphénylphosphino)-1,1'-binaphthyle (BINAP), le triisopropylphosphine, 1,2-bis(diphénylphosphino)éthane (dppe), la tricyclohexylphosphine ($PCy_3$) ; les alkyle et aryle phosphonates choisies parmi, par exemple, le diphénylphosphate, le triphénylphosphate (TPP), le tri(isopropylphényl)phosphate (TIPP), le crésyldiphenyl phosphate (CDP), le tricrésylphosphate (TCP) ; les alkyle et aryle phosphates choisis parmi, par exemple, le di-n-butylphosphate (DBP), le tris-(2-éthylhexyl)-phosphate, le triéthyl phosphate ; les alkyle et aryle phosphinites et phosphonites choisis parmi, par exemple, les méthyldiphénylphosphinite et méthyldiphénylphosphonite ; les azaphosphines choisies parmi, par exemple, le 2,8,9-triisopropyl-2,5,8,9-tetraaza-1-phosphabicyclo[3.3.3]undecane ($BV^{Me}$) et le 2,8,9-triisobutyl-2,5,8,9-tetraaza-1-phosphabicyclo[3.3.3]undecane ($BV^{iBu}$) ; ou
- les bases oxygénées choisies parmi, par exemple, le peroxyde d'hydrogène, le peroxyde de benzoyle, l'oxyde de pyridine (PyO), l'oxyde de N-méthylmorpholine ou le 1-$\lambda^1$-oxidanyl-2,2,6,6-tétraméthylpipéridine.

Des exemples de carbènes N-hétérocycliques sont représentés ci-dessous ;

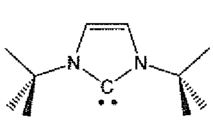
ItBu

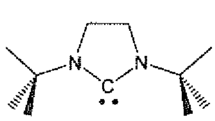
s-ItBu

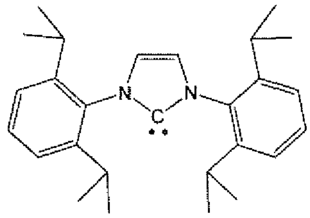
IPr

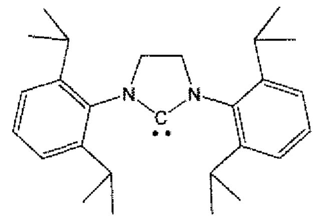
s-IPr

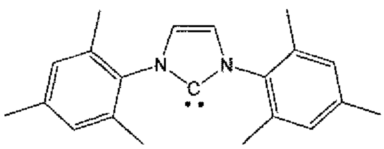
IMes

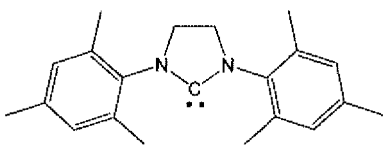
s-IMes

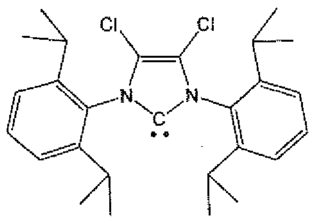
$Cl_2$-IPr

Dans le procédé de l'invention, le catalyseur peut être également (ii) un composé organique ou inorganique de bore choisi parmi, par exemple, le $BF_3$, le $BF_3(Et_2O)$, le $BCl_3$, le triphényl hydroborane, le tricyclohexyl hydroborane,

le $B(C_6F_5)_3$, le B-méthoxy-9-borabicyclo[3.3.1]nonane, le B-benzyl-9-borabicyclo[3.3.1]nonane (B-méthoxy-9BBN), le Me-TBD-BBN$^+$ I$^-$, le Me-TBD-BBN$^+$ $CF_3SO_3^-$, le $(TDB-BBN)_2$, le TBD-BBN-$CO_2$ ou le TBD-BBN-BBN.

Comme indiqué dans le schéma 4 ci-dessous, le $(TDB-BBN)_2$ résulte de la dimérisation de TBD-BBN, le TBD-BBN-$CO_2$ et le TBD-BBN-BBN correspondent à des adduits entre le TBD-BBN et le $CO_2$ ou le 9-BBN.

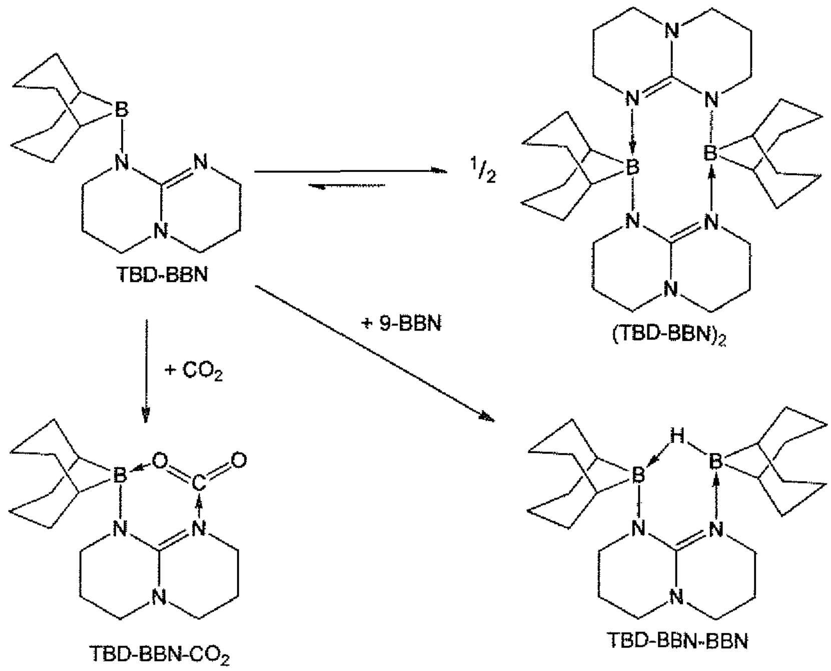


Schéma 4

Le Me-TBD-BBN$^+$ I$^-$, le $(TDB-BBN)_2$, le TBD-BBN-$CO_2$ et le TBD-BBN-BBN peuvent être obtenus, par exemple, selon les protocoles décrits dans la partie expérimentale. Me-TBD-BBN$^+$ $CF_3SO_3^-$ ainsi que Me-TBD-BBN$^+$ X$^-$ dans lequel X$^-$ est choisis parmi le fluor, le chlore et le brome peuvent également être préparés par un protocole proche de celui décrit pour Me-TBD-BBN$^+$ I$^-$.

Dans le procédé de l'invention, le catalyseur peut être, en outre, (iii) un composé organique ou inorganique de l'aluminium choisi parmi, par exemple, $AlCl_3$, $AlBr_3$, l'isopropoxyde d'aluminium ou l'éthanoate d'aluminium.

Selon une variante préférée de l'invention, le catalyseur est (i) une base organique choisie parmi

- les bases azotées, notamment, le triazabicyclodécène (TBD), le N-méthyltriazabicyclodécène (MeTBD), le 1,8-diazabicyclo[5.4.0]undéc-7-ène (DBU), l'arginine ; les phosphazènes choisis parmi le tert-butylimino-tris(diméthylamino)phosphorane (P1-t-Bu), le tert-butylimino-tri(pyrrolidino)phosphorane (BTPP), le tétraméthyl(tris(diméthylamino)phosphoranylidène)phosphorictriamid-Et-imine (P2-Et), le tert-octylimino-tris(diméthylamino)phosphorane (P1-t-Oct) et le 1-tert-butyl-4,4,4-tris(diméthylamino)-2,2-bis[tris(diméthylamino)-phosphoranylidenamino]-2λ5,4λ5-caténadi(phosphazène) (P4-Bu) ;
- les bases phosphorées, notamment, 1,2-bis(diphénylphosphino)éthane (dppe), la tricyclohexylphosphine ($PCy_3$), le 2,8,9-triisopropyl-2,5,8,9-tetraaza-1-phosphabicyclo [3.3.3]undecane ($BV^{Me}$), le 2,8,9-triisobutyl-2,5,8,9-tetraaza-1-phosphabicyclo [3.3.3]undecane ($BV^{iBu}$) ;
- l'oxyde de pyridine (PyO).

Les catalyseurs peuvent, le cas échéant, être immobilisés sur des supports hétérogènes, par exemple, afin d'assurer une séparation facile dudit catalyseur et/ou son recyclage. Lesdits supports hétérogènes peuvent être choisis parmi les supports à base de gel de silice ou de polymères plastiques comme, par exemple, le polystyrène ; les supports carbonés choisis parmi les nanotubes de carbone ; le carbure de silice ; l'alumine ; ou le chlorure de magnésium ($MgCl_2$).

Dans le procédé selon l'invention, la réaction peut se produire sous une pression de $CO_2$, en faisant barboter du $CO_2$ dans le milieu réactionnel ou sous une atmosphère sèche contenant du $CO_2$ (air ambiant séché comprenant, par exemple environ 78% en volume d'azote, 21% en volume d'oxygène, et d'environ de 0,2 à 0,04% en volume de dioxyde de carbone). La réaction peut également se produire en utilisant du $CO_2$ supercritique.

De préférence, la réaction se produit sous une pression de $CO_2$.

La pression du $CO_2$, peut alors être comprise entre 0,2 et 75 bars, de préférence entre 0,2 et 30 bars, plus

préférentiellement entre 1 et 10 bars, bornes incluses.

La température de la réaction peut être comprise entre 20 et 150°C, de préférence entre 20 et 125°C, plus préférentiellement entre 20 et 70°C, bornes incluses.

La durée de la réaction dépend du taux de conversion du hydroborane de formule (II) et de la nature du composé oxyborane de formule (I) voulu. La réaction peut être effectuée pendant une durée de 5 minutes à 300 heures, avantageusement de 2 minutes à 250 heures, de préférence de 10 minutes à 200 heures, bornes incluses.

Le procédé de l'invention, en particulier la réaction entre les différents réactifs, peut avoir lieu dans un ou un mélange d'au moins deux solvant(s) choisi(s) parmi :

- les éthers, de préférence, l'éther diéthylique, ou le THF ;
- les hydrocarbures, de préférence, le benzène, ou le toluène ;
- les solvants azotés, de préférence, la pyridine, ou l'acétonitrile ;
- les sulfoxydes, de préférence, le diméthylesulfoxyde ;
- les halogénures d'alkyle, de préférence, le chloroforme, ou le chlorure de méthylène.

Les différents réactifs utilisés dans le procédé de l'invention (les hydroboranes de formule (II), les (pré-) catalyseurs etc.) sont, en général, des composés commerciaux ou peuvent être préparés par tout procédé connu de l'homme du métier.

La concentration de l'hydroborane de formule (II) est de 0,1 à 2 mol/L, de préférence de 0,3 à 1,5 mol/L, plus préférentiellement de 0,5 à 1,5 mol/L. Cette concentration est calculée sur la base du volume de solvant introduit.

La quantité de catalyseur est de 0,00001 à 1 équivalent molaire, de préférence de 0,0001 à 0,1 équivalent molaire, plus préférentiellement de 0,001 à 0,1 équivalent molaire, bornes incluses, par rapport à l'hydroborane de formule (II).

Comme indiqué précédemment, les composés oxyboranes de formule (I), préparés par le procédé de l'invention, présentent l'avantage de se prêter aisément à différents types de réaction pour conduire à des composés chimiques variés comme des dérivés de méthane, en particulier, des dérivés oxygénés, halogénés ou aminés de méthane notamment l'acide formique, le formaldéhyde, le méthanol, le méthane, l'halogénure de méthyle, la méthylamine. Par exemple, l'hydrolyse des composés de formule (I) dans des conditions connues de l'homme du métier conduit à l'acide formique lorsque Y représente -CHO, au formaldéhyde lorsque Y représente $-CH_2-O-BR^1R^2$ avec $R^1$ et $R^2$ tels que définis ci-dessus, et au méthanol lorsque Y représente $-CH_3$. Les oxyboranes de formule (I) peuvent également réagir avec un acide halogénohydrique choisi HF, HCl, HBr et HI. Par exemple, la réaction des oxyboranes de formule (I) avec HI, dans des conditions connues de l'homme du métier, conduit à l'iodure de méthyle lorsque Y représente $-CH_3$. L'iodure de méthyle peut, à son tour, être soumis à une réaction avec une amine pour conduire à la méthylamine correspondante comme indiqué dans les bases de données REAXYS.

L'invention a donc pour objet un procédé de préparation de l'acide formique, du formaldéhyde, du méthanol, du méthane, de l'halogénure de méthyle, de la méthylamine, éventuellement marqués, comprenant une étape de préparation de composés oxyboranes éventuellement marqués de formule (I) selon l'invention.

La description divulgue l'utilisation des composés oxyboranes de formule (I) obtenus selon le procédé de l'invention, pour la préparation des dérivés de méthane, en particulier, des dérivés oxygénés, halogénés ou aminés de méthane notamment l'acide formique, le formaldéhyde, le méthanol, le méthane, les dérivés halogénure de méthyle et méthylamines.

Les dérivés de méthane ainsi obtenus, peuvent alors être utilisés dans la fabrication de vitamines, de produits pharmaceutiques, de colles, de fibres acryliques et cuirs synthétiques, de pesticides, et d'engrais.

L'invention concerne également un procédé de fabrication de vitamines, de produits pharmaceutiques, de colles, de fibres acryliques, de cuirs synthétiques, de pesticides, et d'engrais, comprenant

- une étape de préparation de composés oxyboranes éventuellement marqués de formule (I) selon l'invention, et
- une étape de préparation de l'acide formique, du formaldéhyde, du méthanol, du méthane, de l'halogénure de méthyle, de la méthylamine, éventuellement marqués, à partir de composés oxyboranes éventuellement marqués de formule (I) préparés selon l'invention.

La description divulgue également un procédé de fabrication de vitamines, de produits pharmaceutiques, de colles, de fibres acryliques, de cuirs synthétiques, de pesticides, et d'engrais, comprenant une étape de préparation des dérivés de méthane, en particulier, des dérivés oxygénés, halogénés ou aminés de méthane, à partir de composés oxyboranes obtenus par le procédé selon l'invention.

Comme déjà indiqué, le procédé selon l'invention conduit à la formation de composés oxyboranes avec un bon voire excellent rendement (allant de 50% à 100%, par exemple), et une bonne sélectivité (pouvant atteindre 100% en un seul type de composé oxyborane). Dans le cas où le catalyseur est supporté, une simple filtration peut permettre

de le récupérer et d'éliminer les éventuels sous-produits à base de bore formés.

Le procédé de l'invention permet de préparer également des composés oxyboranes de formule (I) marqués. Cela constitue un autre objet de l'invention. Les composés oxyboranes marqués correspondent aux composés oxyborane de formule (I) comportant au moins un radiomarqueur/radiotraceur ou un isotope choisi.

Par isotopes on entend, pour un même élément, deux atomes ayant le même nombre de protons (et d'électrons) mais un nombre de neutrons différent. Ayant le même nombre d'électrons et de protons, les propriétés chimiques des isotopes d'un même élément sont presque identiques. Il peut exister, cependant, de légères variations de la vitesse d'une réaction chimique lorsqu'un des atomes d'un réactif est remplacé par un de ses isotopes. En revanche, comme le noyau ne comporte pas le même nombre de neutrons, la masse des atomes varie ce qui peut rendre l'atome instable : c'est pourquoi ils peuvent être radioactifs. Il s'agit alors de radioisotopes. Dans le cadre de l'invention, le terme « isotopes » peut également englober les « radioisotopes ».

Le radiomarquage est le fait d'associer à une molécule ou un composé donnés un isotope qui permettra de suivre l'évolution ou/et la fixation des molécules, par exemple, dans un organe. Le radiotraceur est le ou le(s) élément(s) radioactif(s) présent(s) au sein d'une molécule pour suivre le cheminement de cette substance, par exemple, dans un organe.

Ce procédé peut ainsi permettre l'accès aux composés oxyboranes de formule (I) marqués et à leur produit de réactions. Par exemple, la réaction entre un méthoxyborane marqué et l'acide iodhydrique fourni l'iodométhane marqué correspondant qui est utilisé dans la synthèse des méthylamines marquées (S. C. Choudhry, L. Serico, J. Cupano, Journal of Organic Chemistry, 1989, vol. 54, p. 3755-3757).

L'utilisation de molécules à des fins de traçage, de métabolisation, d'imagerie, etc., est détaillée dans la littérature (U. Pleiss, R. Voges, Synthesis and Applications of Isotopically Labelled Compounds, Volume 7, Wiley-VCH, 2001 ; R. Voges, J. R. Heys, T. Moenius, Preparation of Compounds Labeled with Tritium and Carbon-14, Wiley-VCH: Chippenham (UK) 2009).

La possibilité de former les composés oxyboranes de formule (I) marqués peut être assurée par la disponibilité des réactifs marqués correspondant, par exemple, par :

- les hydroboranes $R^1R^2BH$ marqués $^2H$ (D) ou $^3H$ (T) sont obtenus par deutération de dihydroboranes $(R^1R^2B)_2$ en présence de deutérium moléculaire $D_2$ (C. S. Wei, C. A. Jimenez-Hoyos, M. F. Videa, J. F. Hartwig, M. B. Hall, J. Am. Chem. Soc. 2010, 132, 3078) ;
- les hydroboranes $R^1R^2BH$ marqués $^2H$ (D) ou $^3H$ (T) sont obtenus par échange H/D en présence de deutérium moléculaire $D_2$ et de hydroborane $R^1R^2BH$ (J. Y. Wu, B. Moreau, T. Ritter, J. Am. Chem. Soc. 2009, 131, 12915 ; J. M. Farrell, J. A. Hatnean, D. W. Stephan, J. Am. Chem. Soc. 2012, 134, 15728 ; S. Bontemps, L. Vendier, S. Sabo-Etienne, Angew. Chem. Int. Ed. 2012, 51, 1671) ;
- les hydroboranes $R^1R^2BH$ marqués $^2H$ (D) ou $^3H$ (T) sont obtenus en utilisant $BH_3$(THF) comme réactif boré dans la synthèse du hydroborane, à la place de $BH_3$(THF) (J. M. Brown, G. C. Lloydjones, J. Am. Chem. Soc. 1994, 116, 866) ;
- les hydroboranes $R^1R^2BH$ marqués $^2H$ (D) ou $^3H$ (T) sont obtenus en faisant réagir un halogénure boré de formule $R^1R^2BX$ (X = F, Cl, Br ou I) avec un hydrure métallique marqué, tel que l'hydrure de lithium (LiH) ou le tétrahydruroaluminate de lithium ($LiAlH_4$) (Y. Segawa, Y. Suzuki, M. Yamashita, K. Nozaki, J. Am. Chem. Soc. 2008, 130, 16069 ; Z. P. Lu, Z. H. Cheng, Z. X. Chen, L. H. Weng, Z. H. Li, H. D. Wang, Angew. Chem. Int. Ed. 2011, 50, 12227), les hydrures étant disponibles tous deux en versions deutérée et tritiée (T. A. Kochina, D. V. Vrazhnov, E. N. Sinotova, V. V. Avrorin, M. Y. Katsap, Y. V. Mykhov, Russ J Gen Chem+ 2002, 72, 1222 ; E. A. Shishigin, V. V. Avrorin, T. A. Kochina, E. N. Sinotova, Russ J Gen Chem+ 2005, 75, 152) ;
- le $CO_2$ marqué $^{11}C$ ou $^{14}C$ est la source principale de $^{11}C$ et $^{14}C$ est obtenue par acidification du carbonate de baryum marqué $Ba^{14}CO_3$ (R. Voges, J. R. Heys, T. Moenius, Preparation of Compounds Labeled with Tritium and Carbon-14, Wiley-VCH: Chippenham (UK) 2009).

Selon une variante préférée de l'invention, dans le procédé de préparation de composés oxyboranes marqués de formule (I), le $CO_2$ utilisé est du $CO_2$ marqué dans lequel C représente un isotope $^{11}C$, $^{13}C$ ou $^{14}C$.

Selon une autre variante préférée de l'invention, dans le procédé de préparation de composés oxyboranes marqués de formule (I), l'hydroborane utilisé est un hydroborane marqué de formule $R^1R^2BH$ dans laquelle H représente le deutérium ($^2H$) ou le tritium ($^3H$).

Selon encore une autre variante préférée de l'invention, dans le procédé de préparation de composés oxyboranes marqués de formule (I), le $CO_2$ et l'hydroborane utilisés sont tous les deux marqués : le $CO_2$ est du $CO_2$ marqué dans lequel C représente un isotope $^{11}C$, $^{13}C$ ou $^{14}C$, l'hydroborane est un hydroborane marqué de formule $R^1R^2BH$ dans laquelle H représente le deutérium ($^2H$) ou le tritium ($^3H$).

Les molécules marquées $^{14}C$ ont contribué à de nombreuses avancées dans les sciences du vivant (mécanismes enzymatiques, mécanismes biosynthétiques, biochimie), les sciences de l'environnement (traçage de déchets),

la recherche (élucidation de mécanismes réactionnels) ou encore le diagnostic, la recherche et le développement de nouveaux produits pharmaceutiques et thérapeutiques. Les molécules marquées $^{14}C$ présentent, en effet, un avantage pour les études métaboliques car le $^{14}C$ est facilement détectable et quantifiable en milieu *in vitro* comme *in vivo.*

La source principale de $^{14}C$ est le $^{14}CO_2$ qui est obtenu par acidification du carbonate de baryum $Ba^{14}CO_3$. La mise au point de procédés de synthèse de molécules de base servant à l'élaboration de médicaments est donc primordiale pour produire des principes actifs marqués $^{14}C$ dont le métabolisme pourra ainsi être déterminé (R. Voges, J. R. Heys, T. Moenius, Preparation of Compounds Labeled with Tritium and Carbon-14, Wiley-VCH: Chippenham (UK) 2009**.**

La contrainte majeure limitant la synthèse de molécules marquées $^{14}C$ est la nécessité d'avoir un rendement élevé en produit $^{14}C$ formé par rapport à la quantité de $^{14}CO_2$ utilisée et de reposer sur un nombre restreint d'étapes afin de limiter au maximum les coûts liés à l'utilisation de $Ba^{14}CO_3$ (U. Pleiss, R. Voges, Synthesis and Applications of Isotopically Labelled Compounds, Volume 7, Wiley-VCH, 2001; R. Voges, J. R. Heys, T. Moenius, Preparation of Compounds Labeled with Tritium and Carbon-14, Wiley-VCH: Chippenham (UK) 2009).

Le procédé selon l'invention répond à ces exigences car la pression de travail en $CO_2$ peut être faible, par exemple de 0,2 à 1 bar. En outre, le taux d'incorporation en $CO_2$ (ou rendement par rapport au $CO_2$ introduit) reste élevé, et peut, par exemple, dépasser les 95%.

Les conditions de température, de durée de réaction, de solvant, ainsi que les quantités de réactifs et de catalyseurs mises en oeuvre dans le procédé de préparation des composés oxyboranes marqués de formule (I') sont celles décrites précédemment dans le cadre du procédé de préparation des composés oxyboranes de formule (I).

La description divulgue l'utilisation des composés oxyboranes de formule (I) marqués obtenus selon le procédé de l'invention, pour la préparation des dérivés marqués de méthane, en particulier, des dérivés oxygénés, halogénés ou aminés de méthane notamment l'acide formique, le formaldéhyde, le méthanol, le méthane, l'halogénure de méthyle, la méthylamine.

L'invention a pour objet un procédé de préparation des dérivés marqués de l'acide formique, du formaldéhyde, du méthanol, du méthane, de l'halogénure de méthyle, de la méthylamine, comprenant une étape de préparation de composés oxyboranes marqués de formule (I) selon l'invention.

Les dérivés marqués de méthane ainsi obtenus, peuvent alors être utilisés dans la fabrication de vitamines, de produits pharmaceutiques, de colles, de fibres acryliques et cuirs synthétiques, de pesticides, et d'engrais, par exemple.

L'invention concerne également un procédé de fabrication de vitamines, de produits pharmaceutiques, de colles, de fibres acryliques, de cuirs synthétiques, de pesticides, et d'engrais, comprenant

- une étape de préparation de composés oxyboranes marqués de formule (I) selon l'invention, et
- une étape de préparation de l'acide formique, du formaldéhyde, du méthanol, du méthane, de l'halogénure de méthyle, de la méthylamine, marqués, à partir de composés oxyboranes marqués de formule (I) préparés selon l'invention.

La description divulgue également un procédé de fabrication de vitamines, de produits pharmaceutiques, de colles, de fibres acryliques, de cuirs synthétiques, de pesticides, et d'engrais, par exemple, comprenant une étape de préparation des dérivés marqués de méthane, en particulier, des dérivés oxygénés, halogénés ou aminés de méthane, à partir de composés oxyboranes de formule (I) marqués obtenus par le procédé selon l'invention.

L'invention a, en outre, pour objet un procédé de fabrication de traceurs et de radiotraceurs, caractérisé en ce qu'il comprend

- une étape de préparation de composés oxyboranes marqués de formule (I) selon l'invention, et
- une étape de préparation de l'acide formique, du formaldéhyde, du méthanol, du méthane, de l'halogénure de méthyle, de la méthylamine, à partir de composés oxyboranes marqués de formule (I) préparés selon l'invention.

La description divulgue, en outre, un procédé de fabrication de traceurs et de radiotraceurs, caractérisé en ce qu'il comprend une étape de préparation des dérivés marqués de méthane, en particulier, des dérivés oxygénés, halogénés ou aminés de méthane, à partir de composés oxyboranes de formule (I) marqués obtenus par le procédé selon l'invention.

D'autres avantages et caractéristiques de la présente invention apparaîtront à la lecture des exemples ci-dessous donnés à titre illustratif et non limitatif.

## EXEMPLES

### EXEMPLE 1 :

La réaction catalytique d'hydroboration du $CO_2$ en méthanol, présentée dans le schéma 5 ci-dessous, a été effectuée selon le protocole expérimental suivant.

Sous atmosphère inerte, en boîte à gants, l'hydroborane $R^1R^2BH$ (1 équivalent), le pré-catalyseur (0,0001 à 1 équivalent molaire) et le solvant sont introduits dans un tube de Schlenk qui est ensuite scellé par un robinet J. Young. La concentration en hydroborane dans le mélange réactionnel est d'environ 0,5 $mol.L^{-1}$ (concentration calculée sur la base du volume de solvant introduit). L'ordre d'introduction des réactifs n'a pas d'importance.

Le Schlenk est ensuite mis sous pression de $CO_2$ (de 1 à 3 bar) à l'aide d'une rampe à vide puis est chauffé à une température comprise entre 25 et 100°C jusqu'à la conversion totale du $CO_2$ (de 5 minutes à 72 heures de réaction).

La réaction, une fois terminée, le composé oxyborane résultat est hydrolysé. A cet effet, un volume d'eau égal au volume de solvant est ajouté avec une seringue et le mélange est agité à la température ambiante pendant 1 h. Les produits volatiles sont transférés, sous pression réduite, dans un deuxième tube de Schlenk conduisant à l'obtention d'une solution aqueuse de méthanol.

Un ensemble de résultats est présenté dans le Tableau 1 ci-dessous, donnant des exemples de conversions de $CO_2$ en méthoxyborane et, après hydrolyse, sa conversion en méthanol. A 20 °C, la TOF maximale observée est de 288 $h^{-1}$ (pour $BV^{Me}$ comme catalyseur) et le TON maximal mesuré est de 2014 (avec $BV^{Me}$ comme catalyseur).

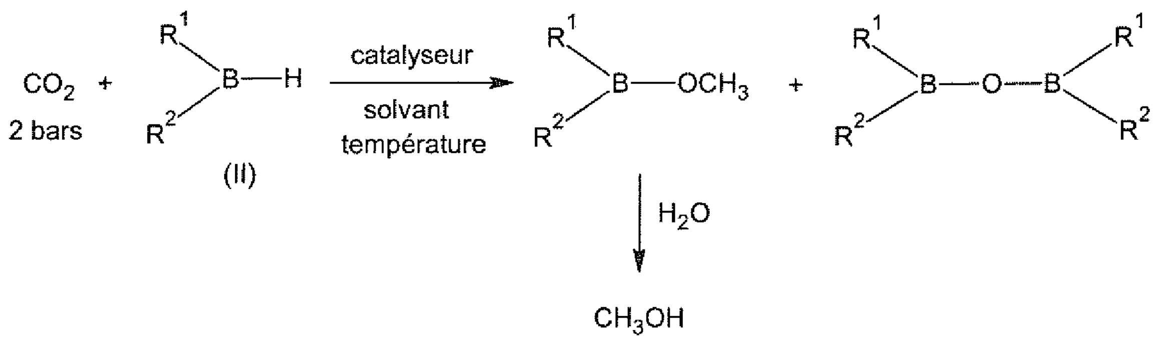


Schéma 5

Différents hydroboranes, catalyseurs, solvants et températures ont été testés pour la réaction.

Les catalyseurs Me-TBD-$BBN^+$ $I^-$, $(TDB\text{-}BBN)_2$, TBD-BBN-$CO_2$ et TBD-BBN-BBN utilisés dans cet exemple ont été préparés selon les protocoles suivants :

- Synthèse de $(TBD\text{-}BBN)_2$

Un ballon de 20 mL équipé d'un barreau aimanté et fermé par un bouchon J. Young est chargé avec de la TBD (163,1 mg, 1,17 mmol, 1 éq), du dimère $(9\text{-}BBN)_2$ (143,0 mg, 0,59 mmol, 0,5 éq) et du tétrahydrofurane (3,5 mL). Le ballon est fermé et la solution est agitée une heure à 70°C. Le mélange réactionnel est refroidi à température ambiante puis le solide est filtré sur fritté et lavé avec du diéthyléther. Un solide blanc est récupéré et séché sous pression réduite pour obtenir le produit $(TBD\text{-}BBN)_2$ avec un rendement de 75% (194,9 mg).

- Synthèse de TBD-BBN-$CO_2$

Un ballon de 20 mL équipé d'un barreau aimanté et fermé par un bouchon J. Young est chargé avec de la $(TBD\text{-}BBN)_2$ (71,0 mg, 0,14 mmol) et du tétrahydrofurane (4 mL). Le mélange réactionnel est mis sous atmosphère de $CO_2$ (1 bar). Le ballon est fermé et la solution est agitée 75 minutes à 100°C. Le solide blanc dans le mélange réactionnel se solubilise progressivement au cours du chauffage. Le mélange réactionnel est refroidi à température ambiante (environ 20°C) puis le solvant est évaporé sous pression réduite afin de récupérer TBD-BBN-$CO_2$ sous la forme d'un solide blanc avec un rendement quantitatif (84,0 mg).

- Synthèse de TBD-BBN-BBN

Un ballon de 20 mL équipé d'un barreau aimanté et fermé par un bouchon J. Young est chargé avec de la $(TBD\text{-}BBN)_2$ (100,0 mg, 0,19 mmol, 1 éq), du dimère $(9\text{-}BBN)_2$ (51,0 mg, 0,21 mmol, 1,1 éq) et du tétrahydrofurane (5 mL). Le ballon est fermé et la solution est agitée 150 minutes à 100°C. Le solide blanc dans le mélange réactionnel se solubilise progressivement au cours du chauffage. Le mélange réactionnel est refroidi à température ambiante puis le solvant est partiellement évaporer du mélange réactionnel jusqu'à environ 0,5 mL. Au cours de l'évaporation du solvant, un solide blanc apparait. Le solide est filtré sur frité et lavé avec du diéthyléther froid (-40°C). Le solide est récupéré et séché sous pression réduite pour obtenir le produit TBD-BBN-BBN avec un rendement de 76% (110,5 mg).

- Synthèse de $MeTBD\text{-}BBN^+ I^-$

Un ballon de 20 mL équipé d'un barreau aimanté et fermé par un bouchon J. Young est chargé avec de la MTBD (53,1 mg, 0,35 mmol, 1 éq) et du tétrahydrofurane (3,5 mL). La solution est agitée et une solution de 9-iodo-9-borabicyclo[3.3.1]nonane 1 M dans l'hexane (350 μL, 0,35 mmol, 1 éq) est ajoutée au mélange réactionnel. Un précipité blanc se forme immédiatement après ajout de la solution de 9-iodo-9-borabicyclo[3.3.1]nonane. Le ballon est fermé et la solution est agitée 30 minutes à température ambiante (environ 20°C). Le solide est filtré sur fritté et lavé avec du diéthyléther. Le solide est récupéré et séché sous pression réduite pour obtenir le produit $MeTBD\text{-}BBN^+ I^-$ avec un rendement de 81% (112,0 mg).

Tableau 1

| $R^1R^2BH$ | Catalyseur | Quantité de catalyseur (éq mol)* | Solvant | Température (°C) | Temps de réaction (h) | Rendement en méthoxyBorane (%) | Rendement en méthanol (%) |
|---|---|---|---|---|---|---|---|
| 9-BBN | TBD | 0,025 | THF | 20 | 27 | 90 | 85 |
| 9-BBN | TBD | 0,01 | THF | 20 | 23 | 89 | 85 |
| 9-BBN | TBD | 0,001 | THF | 20 | 147 | 95 | 90 |
| 9-BBN | Me-TBD | 0,025 | THF | 20 | 7 | 93 | 87 |
| 9-BBN | Me-TBD | 0,01 | THF | 20 | 24 | 81 | 76 |
| 9-BBN | DBU | 0,025 | THF | 20 | 7 | 91 | 85 |
| 9-BBN | DBU | 0,01 | THF | 20 | 17 | 100 | 96 |
| 9-BBN | $(TBD\text{-}BBN)_2$ | 0,012 | THF | 20 | 166 | 80 | 75 |
| 9-BBN | $TBD\text{-}BBN\text{-}CO_2$ | 0,025 | THF | 20 | 7 | 66 | 63 |
| 9-BBN | TBD-BBN-BBN | 0,025 | THF | 70 | 47 | 97 | 92 |
| 9-BBN | $NEt_3$ | 0,025 | THF | 20 | 28 | 98 | 94 |
| 9-BBN | arginine | 0,025 | THF | 20 | 14 | 92 | 86 |
| 9-BBN | DMAP | 0,025 | THF | 70 | 42 | 95 | 89 |
| 9-BBN | dppe | 0,025 | THF | 20 | 24 | 100 | 95 |
| 9-BBN | $PCy_3$ | 0,025 | THF | 20 | 5 | 94 | 89 |
| 9-BBN | P1-t-Bu | 0,025 | THF | 20 | 18 | 100 | 92 |
| 9-BBN | $BV^{Me}$ | 0,025 | THF | 20 | 1 | 87 | 83 |
| 9-BBN | $BV^{Me}$ | 0,01 | THF | 20 | 3 | 95 | 90 |
| 9-BBN | $BV^{Me}$ | 0,005 | THF | 20 | 3 | 95 | 89 |
| 9-BBN | $BV^{Me}$ | 0,005 | THF | 70 | 0,4 | 94 | 89 |
| 9-BBN | $BV^{Me}$ | 0,001 | THF | 20 | 19 | 95 | 90 |
| 9-BBN | $BV^{Me}$ | 0,0001 | THF | 20 | 192 | 95 | 90 |
| 9-BBN | $BV^{Me}$ | 0,0001 | THF | 70 | 144 | 96 | 91 |
| 9-BBN | $BV^{iBu}$ | 0,025 | THF | 20 | 1 | 98 | 94 |
| 9-BBN | ItBu | 0,025 | THF | 20 | 1,5 | 90 | 86 |
| 9-BBN | ItBu | 0,025 | THF | 70 | 0,2 | 100 | 96 |

(suite)

| $R^1R^2BH$ | Catalyseur | Quantité de catalyseur (éq mol)* | Solvant | Température (°C) | Temps de réaction (h) | Rendement en méthoxyBorane (%) | Rendement en méthanol (%) |
|---|---|---|---|---|---|---|---|
| 9-BBN | ItBu | 0,005 | THF | 70 | 0,7 | 98 | 94 |
| catBH | Me-TBD | 0,01 | THF | 20 | 163 | 98 | 93 |
| catBH | TBD-BBN-$CO_2$ | 0,025 | THF | 20 | 155 | 90 | 85 |
| 9-BBN | TBD | 0,025 | benzène | 20 | 170 | 97 | 92 |
| 9-BBN | TBD | 0,025 | toluène | 20 | 170 | 97 | 92 |
| 9-BBN | PyO | 0,025 | THF | 20 | 20 | 96 | 90 |
| 9-BBN | Me-TBD-$BBN^+I^-$ | 0,025 | THF | 20 | 4 | 89 | 85 |

*L'équivalent molaire s'entend par rapport à la quantité d'hydroborane de formule (II).

Ces résultats montrent que, dans les conditions opératoires indiquées dans le Tableau 1, même en présence de catalyseurs et de boranes encombrants, le $CO_2$ peut être transformé en composés méthoxyborane avec de très bons rendements (au moins 66%) et une très bonne sélectivité. Le méthoxyborane fournit, à son tour, après hydrolyse du méthanol avec de très bons rendements (au moins 63%).

**EXEMPLE 2 :**

La réaction catalytique d'hydroboration du $CO_2$ en formoxyborane, présentée dans le schéma 6 ci-dessous, a été effectuée suivant le protocole expérimental indiqué dans l'exemple 1. Les résultats sont présentés dans le Tableau 2 ci-dessous.

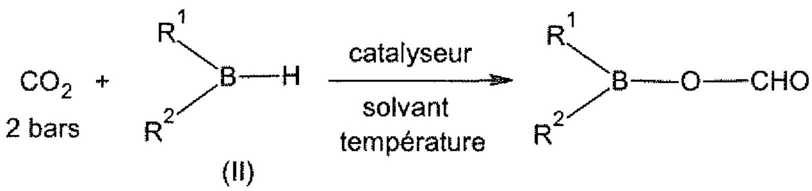

(II)

Schéma 6

Différents catalyseurs ont aussi été testés. Dans tous les cas, le solvant utilisé est le THF et la température de la réaction est de 20°C.

Tableau 2

| $R^1R^2BH$ | Catalyseur | Quantité de catalyseur (éq mol)* | Temps de réaction (h) | Rendement en formoxyborane (%) |
|---|---|---|---|---|
| 9-BBN | TBD | 0,025 | 0,1 | 35 |
| 9-BBN | Me-TBD | 0,025 | 0,1 | 45 |
| 9-BBN | DBU | 0,025 | 0,1 | 32 |
| 9-BBN | $NEt_3$ | 0,025 | 10 | 30 |

*L'équivalent molaire s'entend par rapport à la quantité d'hydroborane de formule (II).

Ces résultats montrent que, dans les conditions opératoires indiquées dans le Tableau 2, le $CO_2$ peut être transformé en composés formoxyborane avec un rendement moyen à bon (30 à 45 %). Parmi les catalyseurs testés, la Me-TBD s'est montrée la plus efficace. Le formoxyborane obtenu, peut fournir, après hydrolyse, de l'acide formique.

**EXEMPLE 3 :**

La réaction catalytique d'hydroboration du $CO_2$ en bis(boryl)acétal, présentée dans le schéma 7 ci-dessous, a été effectuée suivant le protocole expérimental indiqué dans l'exemple 1. Un ensemble de résultats est présenté ci-dessous dans le Tableau 3, montrant des exemples de conversions de $CO_2$ en bis(boryl)acétal.

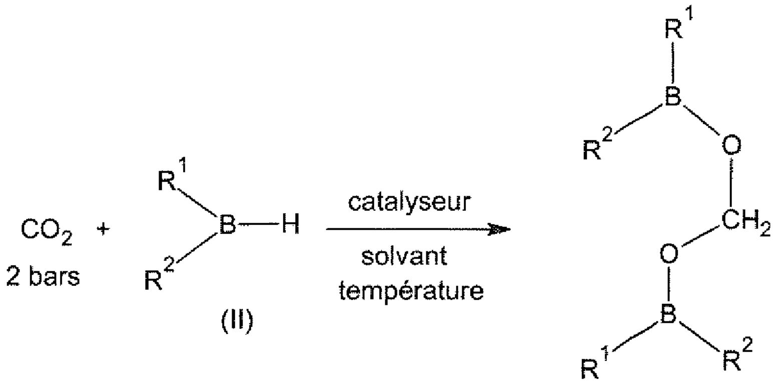


Schéma 7

Différents catalyseurs ont aussi été testés. Dans tous les cas, le solvant utilisé est le THF et la température de la réaction est de 20°C.

Tableau 3

| $R^1R^2BH$ | Catalyseur | Quantité de catalyseur (éq mol)* | Temps de réaction (h) | Rendement en bis(boryl) acétal (%) |
|---|---|---|---|---|
| 9-BBN | TBD | 0,025 | 0,3 | 88 |
| 9-BBN | Me-TBD | 0,025 | 0,3 | 89 |
| 9-BBN | DBU | 0,025 | 0,3 | 79 |
| 9-BBN | $NEt_3$ | 0,025 | 4,5 | 68 |

*L'équivalent molaire s'entend par rapport à la quantité d'hydroborane de formule (II).

Ces résultats montrent que, dans les conditions opératoires indiquées dans le Tableau 3, le $CO_2$ peut être transformé en bis(boryl)acétal avec des rendements bons à excellents (de 68 à 89 %). Parmi les catalyseurs testés, les meilleurs résultats ont été observés avec la triéthylamine et le TBD (le triazabicyclodecène). Le bis(boryl)acétal, peut fournir, après hydrolyse, du formaldéhyde.

Les abréviations utilisées sont :

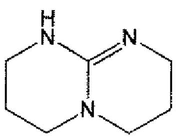
TBD

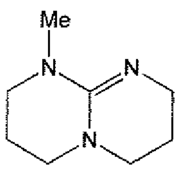
Me-TBD

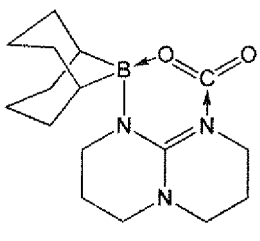
TBD-BBN-$CO_2$

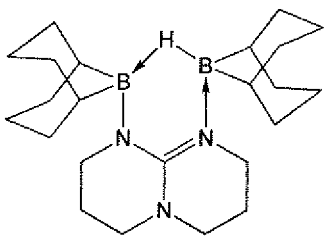
TBD-BBN-BBN

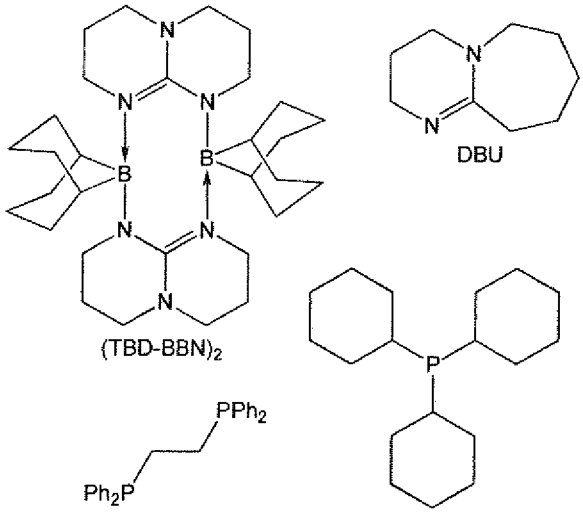

(TBD-BBN)$_2$

DBU

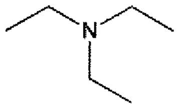

$NEt_3$

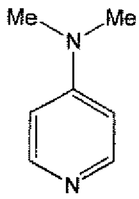

DMAP

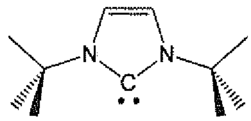

ItBu

dppe

PCy$_3$

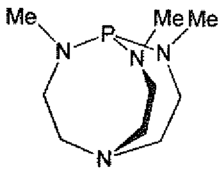

BV$^{Me}$

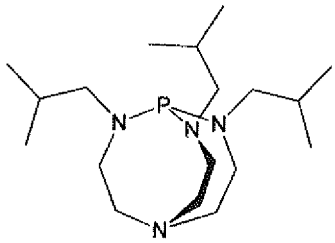

BV$^{iBu}$

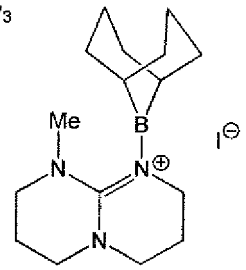

Me-TBD-BBN$^+$ I$^-$

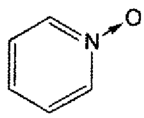

PyO

L'ensemble de ces résultats montrent que la préparation des composés oxyboranes à partir du $CO_2$ et d'un hydroborane, en présence d'une grande variété de catalyseurs se fait dans des conditions de pressions de $CO_2$ et de températures de réaction douces et avec un bon voire très bon rendement et une bonne voire excellente sélectivité (dans certains cas, 100% du composé oxyborane est obtenu). Les composés oxyboranes ainsi obtenus sont suffisamment flexibles pour être convertis, en dérivés de méthane, en particulier, des dérivés oxygénés de méthane notamment l'acide formique, le formaldéhyde et le méthanol.

**EXEMPLE 4 :**

La réaction catalytique d'hydroboration du $CO_2$ en méthanol, présentée dans le schéma 5 ci-dessus, a été effectuée selon le protocole expérimental présenté dans l'exemple 1.

Un ensemble de résultats est présenté dans le Tableau 4 ci-dessous, donnant des exemples de conversions de $CO_2$ en méthoxyborane et, après hydrolyse, sa conversion en méthanol.

Pour un catalyseur donné, en fonction de l'hydroborane associé, la fréquence de rotation (TOF) minimale observée est de 0 $h^{-1}$ (pour IMes comme catalyseur avec le catBH ou le pinBH comme hydroborane par exemple), jusqu'à une TOF maximale observée de 1,1 $h^{-1}$ (pour Me-TBD comme catalyseur avec le 9-BBN comme hydroborane) et le nombre de rotation (TON) maximal mesuré est de 8 (pour Me-TBD comme catalyseur avec le 9-BBN comme hydroborane).

Tableau 4

| $R^1R^2BH$ | Catalyseur | Quantité de catalyseur (éq mol)* | Solvant | Température (°C) | Temps de réaction (h) | Rendement en méthoxy-Borane (%) | Rendement en méthanol (%) |
|---|---|---|---|---|---|---|---|
| 9-BBN | TBD | 0,025 | THF | 20 | 27 | 90 | 85 |
| catBH | TBD | 0,025 | THF | 20 | 72 | 0 | 0 |

(suite)

| $R^1R^2BH$ | Catalyseur | Quantité de catalyseur (éq mol)* | Solvant | Tempé-rature (°C) | Temps de réaction (h) | Rendement en méthoxy-Borane (%) | Rendement en méthanol (%) |
|---|---|---|---|---|---|---|---|
| pinBH | TBD | 0,025 | THF | 20 | 72 | 0 | 0 |
| 9-BBN | Me-TBD | 0,025 | THF | 20 | 7 | 93 | 87 |
| catBH | Me-TBD | 0,01 | THF | 20 | 163 | 98 | 93 |
| pinBH | Me-TBD | 0,025 | THF | 20 | 72 | 0 | 0 |
| 9-BBN | DBU | 0,025 | THF | 20 | 7 | 91 | 85 |
| catBH | DBU | 0,025 | THF | 20 | 0 | 0 | 0 |
| pinBH | DBU | 0,025 | THF | 20 | 0 | 0 | 0 |
| 9-BBN | IMes | 0,025 | THF | 20 | 32 | 91 | 84 |
| catBH | IMes | 0,025 | THF | 20 | 32 | 0 | 0 |
| catBH | IMes | 0,002 | THF | 20 | 32 | 0 | 0 |

Ces résultats montrent que la préparation des composés oxyboranes à partir du $CO_2$ et d'un hydroborane, en présence d'une grande variété de catalyseurs, nécessite un choix judicieux entre le catalyseur et l'hydroborane associé en tenant compte notamment de leur encombrement stérique respectif, du caractère réducteur de l'hydroborane, du caractère nucléophile du catalyseur, de leur solubilité dans le milieu réactionnel.

## Revendications

1. Procédé de préparation de composés oxyboranes de formule (I) :

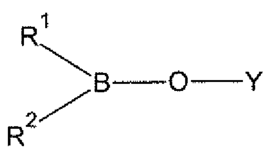

(I)

dans laquelle

- $R^1$ et $R^2$ représentent, indépendamment l'un de l'autre, un atome d'hydrogène, un atome d'halogène, un groupe alkyle linéaire, ramifié ou cyclique, un groupe alcényle, un groupe alcynyle, un groupe aryle, un groupe hétéroaryle, un hétérocycle, un groupe silylé, un groupe siloxy, un groupe amino, un groupe alkoxy, lesdits groupes alkyle, alcényle, alcynyle, aryle, hétéroaryle, hétérocycle, silylé, siloxy, amino et alkoxy étant éventuellement substitués, ou
- $R^1$ et $R^2$, pris ensemble avec l'atome de bore auquel ils sont liés, forment un hétérocycle éventuellement substitué,
- Y représente -CHO, $-CH_2-O-BR^1R^2$ avec $R^1$ et $R^2$ tels que définis ci-dessus, $-CH_3$,
- $R^1$, $R^2$ et Y comportent éventuellement, indépendamment l'un de l'autre, un H, C, N, O, F, Si et/ou S tels que définis ci-dessous,

  - H représente un atome d'hydrogène ($^1H$), le deutérium ($^2H$) ou le tritium ($^3H$),
  - C représente un atome de carbone ($^{12}C$), un isotope $^{11}C$, $^{13}C$ ou $^{14}C$,
  - N représente un atome d'azote ($^{14}N$), un isotope $^{15}N$,
  - O représente un atome d'oxygène ($^{16}O$), un isotope $^{18}O$,
  - F représente un atome de fluor ($^{19}F$), un isotope $^{18}F$,
  - Si représente un atome de silicium ($^{28}Si$), un isotope $^{29}Si$ ou $^{30}Si$,
  - S représente un atome de soufre ($^{32}S$), un isotope $^{33}S$, $^{34}S$ ou $^{36}S$,

**caractérisé en ce que** l'on fait réagir un hydroborane de formule (II) dans laquelle $R^1$, $R^2$ et H sont tels que définis ci-dessus

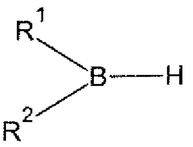

(II)

avec du $CO_2$ dans lequel C et O sont tels que définis ci-dessus, et en présence d'un catalyseur choisi dans le groupe constitué de

i. bases organiques choisies parmi les bases organiques azotées, les bases organiques phosphorées, ou les bases organiques oxygénées

- les bases organiques azotées étant des amines secondaires ou tertiaires choisies parmi, le triazabicyclodécène (TBD), le N-méthyltriazabicyclodécène (MeTBD), le 1,8-diazabicyclo[5.4.0]undéc-7-ène (DBU), la triméthylamine, la triéthyleamine, la pipéridine, la 4-diméthylaminopyridine (DMAP), le 1,4-diazabicyclo[2.2.2]octane (DABCO), la proline, le phénylalanine, un sel de thiazolium, la *N*-diisopropyléthylamine (DIPEA ou DIEA), l'arginine ; les phosphazènes choisis parmi, par exemple, le tert-butylimino-tris(diméthylamino)phosphorane (P1-t-Bu), le tert-butylimino-tri(pyrrolidino)phosphorane (BTPP), le tétraméthyl(tris(diméthylamino)phosphoranylidène)phosphorictriamid-Et-imine (P2-Et), le tert-octylimino-tris(diméthylamino)phosphorane (P1-t-Oct) et le 1-tert-butyl-4,4,4-tris(diméthylamino)-2,2-bis[tris(diméthylamino)-phosphoranylidenamino]-2λ5,4λ5-caténadi(phosphazène) (P4-Bu) ;
- les bases organiques phosphorées étant des alkyles ou aryles phosphines choisis parmi le triphénylphosphine, le 2,2'-bis(diphénylphosphino)-1,1'-binaphthyle (BINAP), le triisopropylphosphine, le 1,2-bis(diphénylphosphino)éthane (dppe), la tricyclohexylphosphine ($PCy_3$) ; les alkyle et aryle phosphonates choisis parmi le diphénylphosphate, le triphénylphosphate (TPP), le tri(isopropylphényl)phosphate (TIPP), le crésyldiphenyl phosphate (CDP), le tricrésylphosphate (TCP) ; les alkyle et aryle phosphates choisis parmi le di-n-butylphosphate (DBP), le tris-(2-éthylhexyl)-phosphate, le triéthyl phosphate ; les alkyle et aryle phosphinites et phosphonites choisis parmi les méthyldiphénylphosphinite et méthyldiphénylphosphonite ; les aza-phosphines choisies parmi le 2,8,9-triisopropyl-2,5,8,9-tetraaza-1-phosphabicyclo[3.3.3]undecane ($BV^{Me}$) et le 2,8,9-triisobutyl-2,5,8,9-tetraaza-1-phosphabicyclo[3.3.3]undecane ($BV^{iBu}$) ; ou
- les bases oxygénées choisies parmi le peroxyde d'hydrogène, le peroxyde de benzoyle, l'oxyde de pyridine (PyO), l'oxyde de N-méthylmorpholine ou le 1-$\lambda^1$-oxidanyl-2,2,6,6-tétraméthylpipéridine ;

ii. composés organiques ou inorganiques de bore choisis parmi le $BF_3$, le $BF_3(Et_2O)$, le $BCl_3$, le triphényl hydroborane, le tricyclohexyl hydroborane, le $B(C_6F_5)_3$, le B-méthoxy-9-borabicyclo[3.3.1]nonane, le B-benzyl-9-borabicyclo[3.3.1]nonane (B-méthoxy-9BBN), le Me-TBD-$BBN^+$ $I^-$, le Me-TBD-$BBN^+$ $CF_3SO_3^-$, le $(TDB\text{-}BBN)_2$, le TBD-BBN-$CO_2$ ou le TBD-BBN-BBN ; ou
iii. composés organiques ou inorganiques de l'aluminium choisis parmi $AlCl_3$, $AlBr_3$, l'isopropoxyde d'aluminium ou l'éthanoate d'aluminium.

2. Procédé selon la revendication 1, **caractérisé en ce que**

- $R^1$ et $R^2$ représentent, indépendamment l'un de l'autre, un atome d'hydrogène, un groupe alkyle linéaire, ramifié ou cyclique, un groupe aryle, un groupe alkoxy, lesdits groupes alkyle, aryle et alkoxy étant éventuellement substitués, ou
- $R^1$ et $R^2$, pris ensemble avec l'atome de bore auquel ils sont liés forment un hétérocycle éventuellement substitué.

3. Procédé selon l'une des revendications 1 ou 2, **caractérisé en ce que**

- $R^1$ et $R^2$, représentent, indépendamment l'un de l'autre, un atome d'hydrogène ; un groupe alkyle linéaire, ramifié ou cyclique choisi parmi les groupes méthyle, éthyle, propyle, butyle, pentyle, hexyle, heptyle, ou leurs isomères ramifiés, cyclohexyle ; un groupe aryle choisi parmi le benzyle ou le phényle ; ou
- $R^1$ et $R^2$, pris ensemble avec l'atome de bore auquel ils sont liés forment un hétérocycle, ledit hétérocycle étant choisi parmi le catBH, le pinBH ou le 9-BBN.

4. Procédé de préparation de composés oxyboranes de formule (I) :

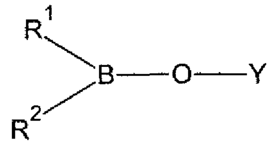

(I)

dans laquelle

- $R^1$ et $R^2$ représentent, indépendamment l'un de l'autre, un atome d'hydrogène, un atome d'halogène, un groupe alkyle linéaire, ramifié ou cyclique, un groupe alcényle, un groupe alcynyle, un groupe aryle, un groupe hétéroaryle, un hétérocycle, un groupe silylé, un groupe siloxy, un groupe amino, un groupe alkoxy, lesdits groupes alkyle, alcényle, alcynyle, aryle, hétéroaryle, hétérocycle, silylé, siloxy, amino et alkoxy étant éventuellement substitués, ou
- $R^1$ et $R^2$, pris ensemble avec l'atome de bore auquel ils sont liés, forment un hétérocycle éventuellement substitué, ledit hétérocycle étant le 9-BBN,
- Y représente -CHO, $-CH_2-O-BR^1R^2$ avec $R^1$ et $R^2$ tels que définis ci-dessus, $-CH_3$,
- $R^1$, $R^2$ et Y comportent éventuellement, indépendamment l'un de l'autre, un H, C, N, O, F, Si et/ou S tels que définis ci-dessous,

  - H représente un atome d'hydrogène ($^1H$), le deutérium ($^2H$) ou le tritium ($^3H$),
  - C représente un atome de carbone ($^{12}C$), un isotope $^{11}C$, $^{13}C$ ou $^{14}C$,
  - N représente un atome d'azote ($^{14}N$), un isotope $^{15}N$,
  - O représente un atome d'oxygène ($^{16}O$), un isotope $^{18}O$,
  - F représente un atome de fluor ($^{19}F$), un isotope $^{18}F$,
  - Si représente un atome de silicium ($^{28}Si$), un isotope $^{29}Si$ ou $^{30}Si$,
  - S représente un atome de soufre ($^{32}S$), un isotope $^{33}S$, $^{34}S$ ou $^{36}S$,

**caractérisé en ce que** l'on fait réagir un hydroborane de formule (II) dans laquelle $R^1$, $R^2$ et H sont tels que définis ci-dessus

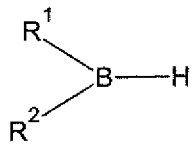

(II)

avec du $CO_2$ dans lequel C et O sont tels que définis ci-dessus, et
en présence d'un catalyseur choisi dans le groupe constitué de bases organiques choisies parmi les bases carbonées, lesdites bases carbonées étant des carbènes N-hétérocycliques issus d'un sel d'imidazolium choisis parmi les sels de 1,3-bis(2,6-diisopropylphenyl)-1H-imidazol-3-ium, 1,3-bis(2,6-diisopropylphenyl)-4,5-dihydro-1H-imidazol-3-ium, 1,3-bis(2,4,6-triméthylphenyl)-1H-imidazol-3-ium, 1,3-bis(2,4,6-triméthylphenyl)-4,5-dihydro-1H-imidazol-3-ium, 4,5-dichloro-1,3-bis(2,6-diisopropylphenyl)-1H-imidazol-3-ium, 1,3-di-tert-butyl-1H-imidazol-3-ium ou carbène ItBu, 1,3-di-tert-butyl-4,5-dihydro-1H-imidazol-3-ium, lesdits sels étant sous la forme de sels de chlorure.

**5.** Procédé selon la revendication 4, **caractérisé en ce que**

- $R^1$ et $R^2$ représentent, indépendamment l'un de l'autre, un atome d'hydrogène, un groupe alkyle linéaire, ramifié ou cyclique, un groupe aryle, un groupe alkoxy, lesdits groupes alkyle, aryle et alkoxy étant éventuellement substitués, ou
- $R^1$ et $R^2$, pris ensemble avec l'atome de bore auquel ils sont liés, forment un hétérocycle éventuellement substitué, ledit hétérocycle étant le 9-BBN.

**6.** Procédé selon l'une des revendications 4 ou 5, **caractérisé en ce que** le catalyseur est le 1,3-di-tert-butyl-1H-imidazol-3-ium ou carbène ItBu.

**7.** Procédé selon l'une quelconque des revendications 1 à 3, **caractérisé en ce que** le catalyseur est (i) une base organique choisie parmi

- les bases azotées, choisies parmi le triazabicyclodécène (TBD), le N-méthyltriazabicyclodécène (MeTBD), le 1,8-diazabicyclo[5.4.0]undéc-7-ène (DBU), l'arginine ; les phosphazènes choisis parmi, le tert-butylimino-tris(dimethylamino)phosphorane (P1-t-Bu), le tert-butylimino-tri(pyrrolidino)phosphorane (BTPP), le tétraméthyl(tris(diméthylamino)phosphoranylidène)phosphorictriamid-Et-imine (P2-Et), le tert-octylimino-tris(diméthylamino)phosphorane (P1-t-Oct) et le 1-tert-butyl-4,4,4-tris(diméthylamino)-2,2-bis[tris(diméthylamino)-phosphoranylidenamino]-2λ5,4λ5-caténadi(phosphazène) (P4-Bu) ;
- les bases phosphorées, choisies parmi 1,2-bis(diphénylphosphino)éthane (dppe), la tricyclohexylphosphine ($PCy_3$), le 2,8,9-triisopropyl-2,5,8,9-tetraaza-1-phosphabicyclo[3.3.3]undecane ($BV^{Me}$), le 2,8,9-triisobutyl-2,5,8,9-tetraaza-1-phosphabicyclo[3.3.3]undecane ($BV^{iBu}$) ; ou
- l'oxyde de pyridine (PyO).

**8.** Procédé selon l'une quelconque des revendications 1 à 7, **caractérisé en ce que** la réaction est effectuée sous une pression de $CO_2$, comprise entre 0,2 et 75 bars, de préférence entre 0,2 et 30 bars, plus préférentiellement entre 1 et 10 bars, bornes incluses.

**9.** Procédé selon l'une quelconque des revendications 1 à 8, **caractérisé en ce que** la réaction est effectuée à une température comprise entre 20 et 150°C, de préférence entre 20 et 125°C, plus préférentiellement entre 20 et 70°C, bornes incluses.

**10.** Procédé selon l'une quelconque des revendications 1 à 9, **caractérisé en ce que** la réaction est effectuée dans un ou un mélange d'au moins deux solvant(s) choisi(s) parmi :

- les éthers, de préférence, l'éther diéthylique, ou le THF ;
- les hydrocarbures, de préférence, le benzène, ou le toluène ;
- les solvants azotés, de préférence, la pyridine, ou l'acétonitrile ;
- les sulfoxydes, de préférence, le diméthylesulfoxyde ;
- les halogénures d'alkyle, de préférence, le chloroforme, ou le chlorure de méthylène.

**11.** Procédé selon l'une quelconque des revendications 1 à 10, **caractérisé en ce que** la concentration du hydroborane de formule (II) est de 0,1 à 2 mol/L, de préférence de 0,3 à 1,5 mol/L, plus préférentiellement de 0,5 à 1,5 mol/L.

**12.** Procédé selon l'une quelconque des revendications 1 à 11, **caractérisé en ce que** la quantité de catalyseur est de 0,00001 à 1 équivalent molaire, de préférence de 0,0001 à 0,1 équivalent molaire, plus préférentiellement de 0,001 à 0,1 équivalent molaire, bornes incluses, par rapport à l'hydroborane de formule (II).

**13.** Procédé de préparation de l'acide formique, du formaldéhyde, du méthanol, du méthane, de l'halogénure de méthyle, de la méthylamine, éventuellement marqués, comprenant une étape de préparation de composés oxyboranes éventuellement marqués de formule (I) selon l'une quelconque des revendications 1 à 12.

**14.** Procédé de fabrication de vitamines, de produits pharmaceutiques, de colles, de fibres acryliques, de cuirs synthétiques, de pesticides, et d'engrais, comprenant

- une étape de préparation de composés oxyboranes éventuellement marqués de formule (I) selon l'une quelconque des revendications 1 à 12, et
- une étape de préparation de l'acide formique, du formaldéhyde, du méthanol, du méthane, de l'halogénure de méthyle, de la méthylamine, éventuellement marqués, à partir de composés oxyboranes éventuellement marqués de formule (I) préparés selon l'une quelconque des revendications 1 à 12.

**15.** Procédé de fabrication de traceurs et de radiotraceurs, **caractérisé en ce qu'**il comprend

- une étape de préparation de composés oxyboranes marqués de formule (I) selon l'une quelconque des revendications 1 à 12, et
- une étape de préparation de l'acide formique, du formaldéhyde, du méthanol, du méthane, de l'halogénure de méthyle, de la méthylamine, à partir de composés oxyboranes marqués de formule (I) préparés selon l'une quelconque des revendications 1 à 12.

## Patentansprüche

1. Verfahren zur Herstellung von Oxyboranverbindungen der Formel (I):

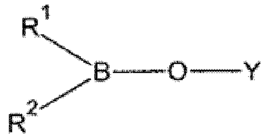

(I)

wobei

- $R^1$ und $R^2$ unabhängig voneinander stehen für ein Wasserstoffatom, ein Halogenatom, eine geradkettige, verzweigte oder zyklische Alkylgruppe, eine Alkenylgruppe, eine Alkinylgruppe, eine Arylgruppe, eine Heteroarylgruppe, einen Heterocyclus, eine silylierte Gruppe, eine Siloxygruppe, eine Aminogruppe, eine Alkoxygruppe, wobei die Alkyl-, Alkenyl-, Alkinyl-, Aryl-, Heteroaryl-, Heterocyclus-, silylierte, Siloxy-, Amino- und Alkoxygruppen gegebenenfalls substituiert sind, oder
- $R^1$ und $R^2$ zusammengenommen mit dem Boratom, an das sie gebunden sind, einen Heterocyclus bilden, der gegebenenfalls substituiert ist,
- Y für -CHO, $-CH_2-O-BR^1R^2$, mit $R^1$ und $R^2$ sowie oben definiert, $-CH_3$ steht,
- $R^1$, $R^2$ und Y gegebenenfalls unabhängig voneinander ein H, C, N, O, F, Si und/oder S so wie unten definiert umfassen,

  - H für ein Wasserstoffatom ($^1H$), Deuterium ($^2H$) oder Tritium ($^3H$) steht,
  - C für ein Kohlenstoffatom ($^{12}C$), ein Isotop $^{11}C$, $^{13}C$ oder $^{14}C$ steht,
  - N für ein Stickstoffatom ($^{14}N$), ein Isotop $^{15}N$ steht,
  - O für ein Sauerstoffatom ($^{16}O$), ein Isotop $^{18}O$ steht,
  - F für ein Fluoratom ($^{19}F$), ein Isotop $^{18}F$ steht,
  - Si für ein Siliziumatom ($^{28}Si$), ein Isotop $^{29}Si$ oder $^{30}Si$ steht,
  - S für ein Schwefelatom ($^{32}S$), ein Isotop $^{33}S$, $^{34}S$ oder $^{36}S$ steht,

**dadurch gekennzeichnet, dass** ein Hydroboran der Formel (II), wobei $R^1$, $R^2$ und H so wie oben definiert sind,

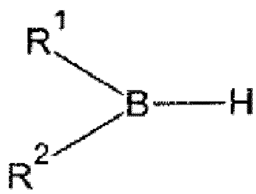

(II)

zum Reagieren gebracht wird mit $CO_2$, wobei C und O so wie oben definiert sind, und
in Gegenwart eines Katalysators, ausgewählt aus der Gruppe bestehend aus

i. organischen Basen, ausgewählt aus den stickstoffhaltigen organischen Basen, den phosphorhaltigen organischen Basen oder den sauerstoffhaltigen organischen Basen,

- wobei die stickstoffhaltigen organischen Basen sekundäre oder tertiäre Amine sind, ausgewählt aus Triazabicyclodecen (TBD), N-Methyltriazabicyclodecen (MeTBD), 1,8-Diazabicyclo[5.4.0]undec-7-en (DBU), Trimethylamin, Triethylamin, Piperidin, 4-Dimethylaminopyridin (DMAP), 1,4-Diazabicyclo[2.2.2]octan (DABCO), Prolin, Phenylalanin, einem Thiazoliumsalz, *N*-Diisopropylethylamin (DIPEA oder DIEA), Arginin; den Phosphazenen, ausgewählt aus zum Beispiel tert-Butylimino-tris(dimethylamino)phosphoran (P1-t-Bu), tert-Butylimino-tri(pyrrolidin)phosphoran (BTPP), Tetramethyl(tris(dimethylamino)phosphoranyliden)phosphorsäuretriamid-Et-imin (P2-Et), tert-Octylimino-tris(dimethylamino)phosphoran (P1-t-Oct) und 1-tert-Butyl-4,4,4-tris(dimethylamino)-2,2-bis[tris(dimethylamino)-phosphoranylidenamino]-2λ5,4λ5-catenadi(phosphazen) (P4-Bu);
- wobei die phosphorhaltigen organischen Basen Alkyl- oder Arylphosphine sind, ausgewählt aus Triphenylphosphin, 2,2'-Bis(diphenylphosphino)-1,1'-binaphthyl (BINAP), Triisopropylphosphin, 1,2-Bis(diphenylphosphino)ethan (dppe), Tricyclohexylphosphin ($PCy_3$); Alkyl- und Arylphosphonate, ausgewählt aus Diphenylphosphat, Triphenylphosphat (TPP), Tri(isopropylphenyl)phosphat (TIPP), Cresyldiphenylphosphat (CDP), Tricresylphosphat (TCP); Alkyl- und Arylphosphate, ausgewählt aus Di-n-butylphosphat (DBP), Tris-(2-ethylhexyl)-phosphat, Triethylphosphat; Alkyl- und Arylphosphinite und -phosphonite, ausgewählt aus Methyldiphenylphosphinit und Methyldiphenylphosphonit; Azaphosphine, ausgewählt aus 2,8,9-Triisopropyl-2,5,8,9-tetraaza-1-phosphabicyclo[3.3.3]undecan ($BV^{Me}$) und 2,8,9-Triisobutyl-2,5,8,9-tetraaza-1-phosphabicyclo[3.3.3]undecan ($BV^{iB}u$); oder

- den sauerstoffhaltigen Basen, ausgewählt aus Wasserstoffperoxid, Benzoylperoxid, Pyridinoxid (PyO), N-Methylmorpholinoxid oder 1-$\lambda^1$-oxidanyl-2,2,6,6-tetramethylpiperidin;

ii. organischen oder anorganischen Borverbindungen, ausgewählt aus $BF_3$, $BF_3(Et_2O)$, $BCl_3$, Triphenylhydroboran, Tricyclohexylhydroboran, $B(C_6F_5)_3$, B-Methoxy-9-borabicyclo[3.3.1]nonan, B-Benzyl-9-borabicyclo[3.3.1]nonan (B-Methoxy-9BBN), Me-TBD-BBN$^+$ I$^-$ , Me-TBD-BBN$^+$ $CF_3SO_3^-$, $(TDB\text{-}BBN)_2$, TBD-BBN-$CO_2$ oder TBD-BBN-BBN; oder

iii) organischen oder anorganischen Aluminiumverbindungen, ausgewählt aus $AlCl_3$, AlBr3, Aluminiumisopropoxid oder Aluminiumethanoat.

**2.** Verfahren nach Anspruch 1, **dadurch gekennzeichnet, dass**

- $R^1$ und $R^2$ unabhängig voneinander stehen für ein Wasserstoffatom, eine geradkettige, verzweigte oder zyklische Alkylgruppe, eine Arylgruppe, eine Alkoxygruppe, wobei die Alkyl-, Aryl- und Alkoxygruppen gegebenenfalls substituiert sind, oder
- $R^1$ und $R^2$ zusammengenommen mit dem Boratom, an das sie gebunden sind, einen Heterocyclus bilden, der gegebenenfalls substituiert ist.

**3.** Verfahren nach einem der Ansprüche 1 oder 2, **dadurch gekennzeichnet, dass**

- $R^1$ und $R^2$ unabhängig voneinander stehen für ein Wasserstoffatom; eine geradkettige, verzweigte oder zyklische Alkylgruppe, ausgewählt aus den Methyl-, Ethyl-, Propyl-, Butyl-, Pentyl-, Hexyl-, Heptylgruppen oder deren verzweigten Isomeren, Cyclohexyl; eine Arylgruppe, ausgewählt aus Benzyl oder Phenyl; oder
- $R^1$ und $R^2$ zusammengenommen mit dem Boratom, an das sie gebunden sind, einen Heterocyclus bilden, wobei der Heterocyclus ausgewählt ist aus catBH, pinBH oder 9-BBN.

**4.** Verfahren zur Herstellung von Oxyboranverbindungen der Formel (I):

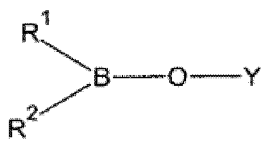

(I)

wobei

- $R^1$ und $R^2$ unabhängig voneinander stehen für ein Wasserstoffatom, ein Halogenatom, eine geradkettige, verzweigte oder zyklische Alkylgruppe, eine Alkenylgruppe, eine Alkinylgruppe, eine Arylgruppe, eine Heteroarylgruppe, einen Heterocyclus, eine silylierte Gruppe, eine Siloxygruppe, eine Aminogruppe, eine Alkoxygruppe, wobei die Alkyl-, Alkenyl-, Alkinyl-, Aryl-, Heteroaryl-, Heterocyclus-, silylierte, Siloxy-, Amino- und Alkoxygruppen gegebenenfalls substituiert sind, oder
- $R^1$ und $R^2$ zusammengenommen mit dem Boratom, an das sie gebunden sind, einen Heterocyclus bilden, der gegebenenfalls substituiert ist, wobei der Heterocyclus 9-BBN ist,
- Y für -CHO, $-CH_2-O-BR^1R^2$, mit $R^1$ und $R^2$ sowie oben definiert, $-CH_3$ steht,
- $R^1$, $R^2$ und Y gegebenenfalls unabhängig voneinander ein H, C, N, O, F, Si und/oder S so wie unten definiert umfassen,

- H für ein Wasserstoffatom ($^1H$), Deuterium ($^2H$) oder Tritium ($^3H$) steht,
- C für ein Kohlenstoffatom ($^{12}C$), ein Isotop $^{11}C$, $^{13}C$ oder $^{14}C$ steht,

- N für ein Stickstoffatom ($^{14}N$), ein Isotop $^{15}N$ steht,
- O für ein Sauerstoffatom ($^{16}O$), ein Isotop $^{18}O$ steht,
- F für ein Fluoratom ($^{19}F$), ein Isotop $^{18}F$ steht,
- Si für ein Siliziumatom ($^{28}Si$), ein Isotop $^{29}Si$ oder $^{30}Si$ steht,
- S für ein Schwefelatom ($^{32}S$), ein Isotop $^{33}S$, $^{34}S$ oder $^{36}S$ steht,

**dadurch gekennzeichnet, dass** ein Hydroboran der Formel (II), wobei $R^1$, $R^2$ und H so wie oben definiert sind,

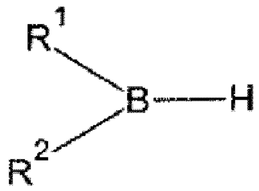

(II)

zum Reagieren gebracht wird mit $CO_2$, wobei C und O so wie oben definiert sind, und in Gegenwart eines Katalysators, ausgewählt aus der Gruppe bestehend aus organischen Basen, ausgewählt aus den kohlenstoffhaltigen Basen, wobei die kohlenstoffhaltigen Basen N-heterozyklische Carbene sind, die aus einem Imidazoliumsalz hervorgegangen sind, ausgewählt aus den Salzen von 1,3-Bis(2,6-diisopropylphenyl)-1H-imidazol-3-ium, 1,3-Bis(2,6-diisopropylphenyl)-4,5-dihydro-1H-imidazol-3-ium, 1,3-Bis(2,4,6-trimethylphenyl)-1H-imidazol-3-ium, 1,3-Bis(2,4,6-trimethylphenyl)-4,5-dihydro-1H-imidazol-3-ium, 4,5-Dichlor-1,3-bis(2,6-diisopropylphenyl)-1H-imidazol-3-ium, 1,3-Di-tert-butyl-1H-imidazol-3-ium oder ItBu-Carben, 1,3-Di-tert-butyl-4,5-dihydro-1H-imidazol-3-ium, wobei die Salze in der Form von Chloridsalzen vorliegen.

5. Verfahren nach Anspruch 4, **dadurch gekennzeichnet, dass**

   - $R^1$ und $R^2$ unabhängig voneinander stehen für ein Wasserstoffatom, eine geradkettige, verzweigte oder zyklische Alkylgruppe, eine Arylgruppe, eine Alkoxygruppe, wobei die Alkyl-, Aryl- und Alkoxygruppen gegebenenfalls substituiert sind, oder
   - $R^1$ und $R^2$ zusammengenommen mit dem Boratom, an das sie gebunden sind, einen Heterocyclus bilden, der gegebenenfalls substituiert ist, wobei der Heterocyclus 9-BBN ist.

6. Verfahren nach einem der Ansprüche 4 oder 5, **dadurch gekennzeichnet, dass** der Katalysator 1,3-Di-tert-butyl-1H-imidazol-3-ium oder ItBu-Carben ist.

7. Verfahren nach einem der Ansprüche 1 bis 3, **dadurch gekennzeichnet, dass** der Katalysator (i) eine organische Base ist, ausgewählt aus

   - den stickstoffhaltigen Basen, ausgewählt aus Triazabicyclodecen (TBD), N-Methyltriazabicyclodecen (MeTBD), 1,8-Diazabicyclo[5.4.0]undec-7-en (DBU), Arginin; den Phosphazenen, ausgewählt aus tert-Butylimino-tris(dimethylamino)phosphoran (P1-t-Bu), tert-Butylimino-tri(pyrrolidin)phosphoran (BTPP), Tetramethyl(tris(dimethylamino)phosphoranyliden)phosphorsäuretriamid-Et-imin (P2-Et), tert-Octylimino-tris(dimethylamino)phosphoran (P1-t-Oct) und 1-tert-Butyl-4,4,4-tris(dimethylamino)-2,2-bis[tris(dimethylamino)-phosphoranylidenamino]-2λ5,4λ5-catenadi(phosphazen) (P4-Bu);
   - den phosphorhaltigen Basen, ausgewählt aus 1,2-Bis(diphenylphosphino)ethan (dppe), Tricyclohexylphosphin ($PCy_3$), 2,8,9-Triisopropyl-2,5,8,9-tetraaza-1-phosphabicyclo[3.3.3]undecan ($BV^{Me}$), 2,8,9-Triisobutyl-2,5,8,9-tetraaza-1-phosphabicyclo[3.3.3]undecan ($BV^{iB}u$); oder
   - Pyridinoxid (PyO).

8. Verfahren nach einem der Ansprüche 1 bis 7, **dadurch gekennzeichnet, dass** die Reaktion unter einem $CO_2$-Druck im Bereich zwischen 0,2 und 75 Bar, vorzugsweise zwischen 0,2 und 30 Bar, stärker bevorzugt zwischen 1 und 10 Bar, Grenzwerte eingeschlossen, vorgenommen wird.

9. Verfahren nach einem der Ansprüche 1 bis 8, **dadurch gekennzeichnet, dass** die Reaktion bei einer Temperatur im Bereich zwischen 20 und 150 °C, vorzugsweise zwischen 20 und 125 °C, stärker bevorzugt zwischen 20 und 70 °C, Grenzwerte eingeschlossen, vorgenommen wird.

**10.** Verfahren nach einem der Ansprüche 1 bis 9, **dadurch gekennzeichnet, dass** die Reaktion in einem, oder einem Gemisch aus mindestens zwei Lösungsmittel(n) vorgenommen wird, ausgewählt aus:

- Ethern, vorzugsweise Diethylether oder THF;
- Kohlenwasserstoffen, vorzugsweise Benzol oder Toluol;
- stickstoffhaltigen Lösungsmitteln, vorzugsweise Pyridin oder Acetonitril;
- Sulfoxiden, vorzugsweise Dimethylsulfoxid;
- Alkylhalogeniden, vorzugsweise Chloroform oder Methylenchlorid.

**11.** Verfahren nach einem der Ansprüche 1 bis 10, **dadurch gekennzeichnet, dass** die Konzentration des Hydroborans der Formel (II) von 0,1 bis 2 mol/L, vorzugsweise von 0,3 bis 1,5 mol/L, stärker bevorzugt von 0,5 bis 1,5 mol/L beträgt.

**12.** Verfahren nach einem der Ansprüche 1 bis 11, **dadurch gekennzeichnet, dass** bezogen auf das Hydroboran der Formel (II) die Katalysatormenge von 0,00001 bis 1 Moläquivalent, vorzugsweise von 0,0001 bis 0,1 Moläquivalent, stärker bevorzugt von 0,001 bis 0,1 Moläquivalent, Grenzwerte eingeschlossen, beträgt.

**13.** Verfahren zur Herstellung von Ameisensäure, Formaldehyd, Methanol, Methan, Methylhalogenid, Methylamin, die gegebenenfalls markiert sind, umfassend einen Schritt des Herstellens von gegebenenfalls markierten Oxyboranverbindungen der Formel (I) nach einem der Ansprüche 1 bis 12.

**14.** Verfahren zur Herstellung von Vitaminen, pharmazeutischen Produkten, Klebern, Acrylfasern, künstlichen Ledern, Pestiziden und Düngern, umfassend:

- einen Schritt des Herstellens von gegebenenfalls markierten Oxyboranverbindungen der Formel (I) nach einem der Ansprüche 1 bis 12, und
- einen Schritt des Herstellens der Ameisensäure, des Formaldehyds, des Methanols, des Methans, des Methylhalogenids, des Methylamins, die gegebenenfalls markiert sind, ausgehend von gegebenenfalls markierten Oxyboranverbindungen der Formel (I), die nach einem der Ansprüche 1 bis 12 hergestellt wurden.

**15.** Verfahren zur Anfertigung von Markierungen und radioaktiven Markierungen, **dadurch gekennzeichnet, dass** es umfasst

- einen Schritt des Herstellens von markierten Oxyboranverbindungen der Formel (I) nach einem der Ansprüche 1 bis 12, und
- einen Schritt des Herstellens der Ameisensäure, des Formaldehyds, des Methanols, des Methans, des Methylhalogenids, des Methylamins, ausgehend von markierten Oxyboranverbindungen der Formel (I), die nach einem der Ansprüche 1 bis 12 hergestellt wurden.

## Claims

**1.** Method for preparing oxyborane compounds of formula (I):

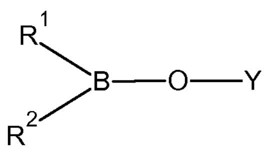

(I)

wherein

- $R^1$ and $R^2$ represent, independently from one another, a hydrogen atom, a halogen atom, a linear, branched or cyclic alkyl group, an alkenyl group, an alkynyl group, an aryl group, a heteroaryl group, a heterocyclic compound, a silyl group, a siloxy group, an amino group, an alkoxy group, where said alkyl, alkenyl, alkynyl aryl, heteroaryl, heterocyclic, silyl, siloxy, amino and alkoxy groups being optionally substituted, or
- $R^1$ and $R^2$, taken together with the boron atom to which they are bound, form a heterocycle optionally substituted,

- Y represents -CHO, $-CH_2-O-BR^1R^2$ where $R^1$ and $R^2$ are as defined above, $-CH_3$,
- $R^1$, $R^2$ and Y optionally include, independently from one another, a H, C, N, O, F, Si and/or S as defined above.

  - H represents a hydrogen atom ($^1H$), deuterium ($^2H$) or tritium ($^3H$),
  - C represents a carbon atom ($^{12}C$), an isotope $^{11}C$, $^{13}C$ or $^{14}C$,
  - N represents a nitrogen atom ($^{14}N$), an isotope $^{15}N$,
  - O represents an oxygen atom ($^{16}O$), an isotope $^{18}O$,
  - F represents a fluorine atom ($^{19}F$), an isotope $^{18}F$,
  - Si represents a silicon atom ($^{28}Si$), an isotope $^{29}Si$ or $^{30}Si$,
  - S represents a sulphur atom ($^{32}S$), an isotope $^{33}S$, $^{34}S$ or $^{36}S$,

**characterised in that** a reaction takes place between a hydroborane of formula (II), in which $R^1$, $R^2$ and H are as defined above

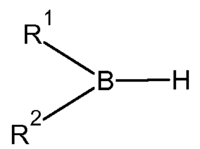

(II)

and $CO_2$, in which C and O are as defined above, and in the presence of a catalyst chosen from the group that includes:

i. organic bases chosen among nitrogenous organic bases, phosphorous organic bases, or oxygen organic bases

- nitrogenous organic bases being secondary or tertiary amines chosen among triazabicyclodecene (TBD), N-methyltriazabicyclodecene (MeTBD), 1,8-diazabicyclo[5.4.0]undec-7-ene (DBU), trimethylamine, triethylamine, piperidine, 4-dimethylaminopyridine (DMAP), 1,4-diazabicyclo[2.2.2]octane (DABCO), proline, phenylalanine, a thiazolium salt, *N*-diisopropylethylamine (DIPEA or DIEA), arginine ; the phosphazenes chosen from, for example, tert-butylimino-tris(dimethylamino)phosphorane (P1-t-Bu), tert-butylimino-tris(pyrrolidino)phosphorane (BTPP), tetramethyl(tris(dimethylamino)phosphanylidene)phosphorictriamid-Et-imine (P2-Et), tert-octylimino-tris(dimethylamino)phosphorane (P1-t-Oct) and 1-tert-butyl-4,4,4-tris(dimethylamino)-2,2-bis[tris(dimethylamino)-phosphoranylidenamino]-2λ5,4λ5-catenadi(phosphazene) (P4-Bu);
- phosphorous organic bases being alkyl or aryl phosphines chosen from triphenylphosphine, 2,2'-bis(diphenylphosphino)-1,1'-binaphthyl (BINAP), triisopropylphosphine, 1,2-bis(diphenylphosphino)ethane (dppe), tricyclohexylphosphine ($PCy_3$); alkyl and aryl phosphonates chosen from diphenyl phosphate, triphenyl phosphate (TPP), tri(isopropylphenyl)phosphate (TIPP), cresyl diphenyl phosphate (CDP), tricresyl phosphate (TCP); alkyl and aryl phosphates chosen from di-n-butyl phosphate (DBP), le tris-(2-ethylhexyl)-phosphate, triethyl phosphate; alkyl and aryl phosphinites and phosphonites chosen from methyldiphenylphosphinite and methyldiphenylphosphonite ; aza-phosphines chosen from 2,8,9-triisopropyl-2,5,8,9-tetraaza-1-phosphabicyclo[3.3.3]undecane ($BV^{Me}$) and 2,8,9-triisobutyl-2,5,8,9-tetraaza-1-phosphabicyclo[3.3.3]undecane ($BV^{iBu}$); or
- oxygen bases chosen from hydrogen peroxide, benzoyl peroxide, pyridine oxide (PyO), N-methylmorpholine oxide or 1-$\lambda^1$-oxidanyl-2,2,6,6-tetramethylpiperidine;

ii. organic or inorganic boron compounds chosen from $BF_3$, $BF_3(Et_2O)$, $BCl_3$, triphenyl hydroborane, tricyclohexyl hydroborane, $B(C_6F_5)_3$, B-methoxy-9-borabicyclo[3.3.1]nonane, B-benzyl-9-borabicyclo[3.3.1]nonane (B-methoxy-9BBN), Me-TBD-$BBN^+$ $I^-$, Me-TBD-$BBN^+$ $CF_3SO_3^-$, $(TDB\text{-}BBN)_2$, TBD-BBN-$CO_2$ or TBD-BBN-BBN; or
iii. organic or inorganic aluminium compounds chosen from $AlCl_3$, $AlBr_3$, aluminium isopropoxide or aluminium ethanoate.

**2.** Method according to claim 1, **characterised in that**

- $R^1$ and $R^2$ represent, independently from one another, a hydrogen atom, a linear, branched or cyclic alkyl group, an aryl group, an alkoxy group, where said alkyl, aryl and alkoxy groups can be substituted, or
- $R^1$ and $R^2$, taken together with the boron atom to which they are bound, form a heterocycle that can be

substituted.

3. Method according to claim 1 or 2, **characterised in that**

- $R^1$ and $R^2$ represent, independently from one another, a hydrogen atom; a linear, branched or cyclic alkyl group chosen among the methyl, ethyl, propyl, butyl, pentyl, hexyl, heptyl groups or their branched isomers, cyclohexyl: an aryl group chosen from benzyl or phenyl; or
- $R^1$ and $R^2$, taken together with the boron atom to which they are bound, form a heterocycle, where said heterocycle is chosen from catBH, pinBH 9-BBN.

4. Method for preparing oxyborane compounds of formula (I):

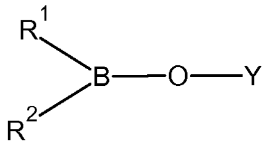

(I)

wherein

- $R^1$ and $R^2$ represent, independently from one another, a hydrogen atom, a halogen atom, a linear, branched or cyclic alkyl group, an alkenyl group, an alkynyl group, an aryl group, a heteroaryl group, a heterocyclic compound, a silyl group, a siloxy group, an amino group, an alkoxy group, where said alkyl, alkenyl, alkynyl aryl, heteroaryl, heterocyclic, silyl, siloxy, amino and alkoxy groups being optionally substituted, or
- $R^1$ and $R^2$, taken together with the boron atom to which they are bound, form a heterocycle optionally substituted, where said heterocycle is 9-BBN.
- Y represents -CHO, $-CH_2-O-BR^1R^2$ where $R^1$ and $R^2$ are as defined above, $-CH_3$,
- $R^1$, $R^2$ and Y can include, independently from one another, a H, C, N, O, F, Si and/or S such as defined above.

  - H represents a hydrogen atom ($^1H$), deuterium ($^2H$) or tritium ($^3H$),
  - C represents a carbon atom ($^{12}C$), an isotope $^{11}C$, $^{13}C$ or $^{14}C$,
  - N represents a nitrogen atom ($^{14}N$), an isotope $^{15}N$,
  - O represents an oxygen atom ($^{16}O$), an isotope $^{18}O$,
  - F represents a fluorine atom ($^{19}F$), an isotope $^{18}F$,
  - Si represents a silicon atom ($^{28}Si$), an isotope $^{29}Si$ or $^{30}Si$,
  - S represents a sulphur atom ($^{32}S$), an isotope $^{33}S$, $^{34}S$ or $^{36}S$,

**characterised in that** a reaction takes place between a hydroborane of formula (II), in which $R^1$, $R^2$ and H are as defined above

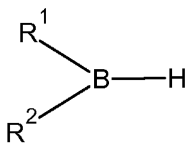

(II)

and $CO_2$, in which C and O are as defined above, and
in the presence of a catalyst chosen from the group made of organic bases chosen among carbon bases, where said carbon bases are N-heterocyclic carbenes derived for a imidazolium salt chosen from the salts of 1,3-bis(2,6-diisopropylphenyl)-1H-imidazol-3-ium, 1,3-bis(2,6-diisopropylphenyl)-4,5-dihydro-1H-imidazol-3-ium, 1,3-bis(2,4,6-trimethylphenyl)-1H-imidazol-3-ium, 1,3-bis(2,4,6-trimethylphenyl)-4,5-dihydro-1H-imidazol-3-ium, 4,5-dichloro-1,3-bis(2,6-diisopropylphenyl)-1H-imidazol-3-ium, 1,3-di-tert-butyl-1H-imidazol-3-ium or carbene ItBu, 1,3-di-tert-butyl-4,5-dihydro-1H-imidazol-3-ium, where said salts are in the form of chloride salts.

5. Method according to claim 4, **characterised in that**

- $R^1$ and $R^2$ represent, independently from one another, a hydrogen atom, a linear, branched or cyclic alkyl group, an aryl group, an alkoxy group, where said alkyl, aryl and alkoxy groups are optionally substituted, or
- $R^1$ and $R^2$, taken together with the boron atom to which they are bound, form a heterocycle optionally substituted, where said heterocycle is 9-BBN.

**6.** Method according to claim 4 or 5, **characterised in that** the catalyst is 1,3-di-tert-butyl-1H-imidazol-3-ium or carbene ItBu.

**7.** Method according to any of the claims 1 to 3, **characterised in that** the catalyst is (i) an organic base chosen from

- nitrogenous bases chosen among triazabicyclodecene (TBD), N-methyltriazabicyclodecene (MeTBD), 1,8-diazabicyclo[5.4.0]undec-7-ene (DBU), arginine; the phosphazenes chosen from, for example, tert-butylimino-tris(dimethylamino)phosphorane (P1-t-Bu), tert-butylimino-tris(pyrrolidino)phosphorane (BTPP), tetramethyl(tris(dimethylamino)phosphanylidene)phosphorictriamid-Et-imine (P2-Et), tert-octylimino-tris(dimethylamino)phosphorane (P1-t-Oct) and 1-tert-butyl-4,4,4-tris(dimethylamino)-2,2-bis[tris(dimethylamino)-phosphoranylidenamino]-2λ5,4λ5-catenadi(phosphazene) (P4-Bu);
- phosphorous bases, chosen from 1,2-bis(diphenylphosphino)ethane (dppe), tricyclohexylphosphine ($PCy_3$), 2,8,9-triisopropyl-2,5,8,9-tetraaza-1-phosphabicyclo[3.3.3]undecane ($BV^{Me}$), 2,8,9-triisobutyl-2,5,8,9-tetraaza-1-phosphabicyclo[3.3.3]undecane ($BV^{iBu}$); or
- pyridine oxide (PyO).

**8.** Method according to any of the claims 1 to 7, **characterised in that** the reaction is conducted at a $CO_2$ pressure between 0.2 and 75 bars, preferentially between 0.2 and 30 bars, and more preferentially between 1 and 10 bars, limits included.

**9.** Method according to any of the claims 1 to 8, **characterised in that** the reaction is conducted at a temperature between 20 and 150°C preferentially between 20 and 125°C, and more preferentially between 20 and 70°C, limits included.

**10.** Method according to any of the claims 1 to 9, **characterised in that** the reaction is conducted in one, or in a mixture of at least two solvent(s) chosen from:

- ethers, preferentially diethyl ether, or THF;
- hydrocarbons, preferentially benzene or toluene;
- nitrogenous solvents, preferentially pyridine or acetonitrile;
- sulfoxides, preferentially dimethlyl sulfoxide;
- alkyl halides, preferentially chloroform, or methylene chloride.

**11.** Method according to any of the claims 1 to 10, **characterised in that** the concentration in hydroborane of formula (II) is between 0.1 to 2 mol/L, preferentially between 0.3 to 1.5 mol/L, and more preferentially from 0.5 to 1.5 mol/L.

**12.** Method according to any of the claims 1 to 11, **characterised in that** the quantity of catalyst is from 0.00001 to 1 molar equivalent, preferentially from 0.0001 to 0.1 molar equivalent, and more preferentially from 0.001 to 0.1 molar equivalent, limits included, with respect to the hydroborane of formula (II).

**13.** Method for preparing formic acid, formaldehyde, methanol, methane, methyl halide, methylamine, optionally labeled, including a step for the preparation of oxyborane compounds, optionally labeled, of formula (I) according to any of the claims 1 to 12.

**14.** Method for producing vitamins, pharmaceutical products, adhesives, acrylic fibres, synthetic leathers, pesticides and fertiliser, including:

- a step for the preparation of oxyborane compounds, optionally labeled, of formula (I) according to the claims 1 to 12, and
- a step for the preparation of formic acid, formaldehyde, methanol, methane, methyl halide, methylamine, optionally labeled, from oxyborane compounds, optionally labeled, of formula (I) prepared according to any of the claims 1 to 12.

**15.** Method for producing tracers and radioactive tracers, **characterised in that** it includes

- a step for the preparation of labeled oxyborane compounds of formula (I) according to the claims 1 to 12, and
- a step for the preparation of formic acid, formaldehyde, methanol, methane, methyl halide, methylamine from marked oxyborane compounds of formula (I) prepared according to any of the claims 1 to 12.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **E. E. BENSON ; C. P. KUBIAK ; A. J. SATHRUM ; J. M. SMIEJA.** *Chem. Soc. Rev.,* 2009, vol. 38, 89
- **Y. IZUMI.** *Coord. Chem. Rev.,* 2013, vol. 257, 171
- **P. G. JESSOP ; T. IKARIYA ; R. NOYORI.** *Chem. Rev.,* 1995, vol. 95, 259
- **S. N. RIDUAN ; Y. G. ZHANG ; J. Y. YING.** *Angew. Chem. Int. Ed.,* 2009, vol. 48, 3322
- **A. BERKEFELD ; W. E. PIERS ; M. PARVEZ.** *J. Am. Chem. Soc.,* 2010, vol. 132, 10660
- **T. MATSUO ; H. KAWAGUCHI.** *J. Am. Chem. Soc.,* 2006, vol. 128
- **S. CHAKRABORTY ; J. ZHANG ; J. A. KRAUSE ; H. R. GUAN.** *J. Am. Chem. Soc.,* 2010, vol. 132, 8872
- **S. CHAKRABORTY ; Y. J. PATEL ; J. A. KRAUSE ; H. R. GUAN.** *Polyhedron,* 2012, vol. 32, 30
- **S. CHAKRABORTY ; J. ZHANG ; Y. J. PATEL ; J. A. KRAUSE ; H. R. GUAN.** *Inorg. Chem.,* 2013, vol. 52, 37
- **S. BONTEMPS ; L. VENDIER ; S. SABO-ETIENNE.** *Angew. Chem. Int. Ed.,* 2012, vol. 51, 1671
- **M. J. SGRO ; D. W. STEPHAN.** *Angew. Chem. Int. Ed.,* 2012, vol. 51, 11343
- **G. MÉNARD ; D. W. STEPHAN.** *J. Am. Chem. Soc.,* 2010, vol. 132, 1796
- **F. HUANG et al.** *Inorganic Chemistry,* 2011, vol. 50 (8), 3816-3825
- **S. C. CHOUDHRY ; L. SERICO ; J. CUPANO.** *Journal of Organic Chemistry,* 1989, vol. 54, 3755-3757
- **U. PLEISS ; R. VOGES.** Synthesis and Applications of Isotopically Labelled Compounds. Wiley-VCH, 2001, vol. 7
- **R. VOGES ; J. R. HEYS ; T. MOENIUS.** Preparation of Compounds Labeled with Tritium and Carbon-14. Wiley-VCH, 2009
- **C. S. WEI ; C. A. JIMENEZ-HOYOS ; M. F. VIDEA ; J. F. HARTWIG ; M. B. HALL.** *J. Am. Chem. Soc.,* 2010, vol. 132, 3078
- **J. Y. WU ; B. MOREAU ; T. RITTER.** *J. Am. Chem. Soc.,* 2009, vol. 131, 12915
- **J. M. FARRELL ; J. A. HATNEAN ; D. W. STEPHAN.** *J. Am. Chem. Soc.,* 2012, vol. 134, 15728
- **J. M. BROWN ; G. C. LLOYDJONES.** *J. Am. Chem. Soc.,* 1994, vol. 116, 866
- **Y. SEGAWA ; Y. SUZUKI ; M. YAMASHITA ; K. NOZAKI.** *J. Am. Chem. Soc.,* 2008, vol. 130, 16069
- **Z. P. LU ; Z. H. CHENG ; Z. X. CHEN ; L. H. WENG ; Z. H. LI ; H. D. WANG.** *Angew. Chem. Int. Ed.,* 2011, vol. 50, 12227
- **T. A. KOCHINA ; D. V. VRAZHNOV ; E. N. SINOTOVA ; V. V. AVRORIN ; M. Y. KATSAP ; Y. V. MYKHOV.** *Russ J Gen Chem+,* 2002, vol. 72, 1222
- **E. A. SHISHIGIN ; V. V. AVRORIN ; T. A. KOCHINA ; E. N. SINOTOVA.** *Russ J Gen Chem+,* 2005, vol. 75, 152